# EUROPEAN PATENT APPLICATION

(11) **EP 1 619 247 A1**
(43) Date of publication of application: **25.01.2006**
(21) Application number: 04729258.6
(22) Date of filing: 23.04.2004
(51) Int. Cl.: C12N 15/00, C12P 21/02, C12M 1/00

(54) **HIGH THROUGHPUT SYNTHESIS SYSTEM AND SYNTHESIZER FOR AUTOMATICALLY PERFORMING THE SYSTEM**

(30) Priority: 25.04.2003 JP 2003122930; 29.07.2003 JP 2003281500
(71) Applicant: CellFree Sciences Co., Ltd., Yokohama-shi, Kanagawa 230-0046 (JP)
(72) Inventor: ENDO, Yaeta, Matsuyama-shi,Ehime 7918016 (JP); SAWASAKI, Tatsuya, Matsuyama-shi, Ehime 7900811 (JP); OGASAWARA, Tomio, Iyo-shi, Ehime 7993104 (JP); MORISHITA, Riyo, c/o Cell Free Sciences Co., Ltd., Yokohama-shi, Kanagawa 2300046 (JP); SAEKI, Mihoro, c/o Cell Free Sciences Co., Ltd., Yokohama-shi, Kanagawa 2300046 (JP); SATO, Tomohisa, c/o Cell Free Sciences Co., Ltd., Yokohama-shi, Kanagawa 2300046 (JP); KITAMOTO, Aya, c/o Cell Free Sciences Co., Ltd., Yokohama-shi, Kanagawa 2300046 (JP)
(74) Representative: Krauss, Jan
(86) International application number: PCT/JP2004/005912
(87) International publication number: WO 2004/097014

(57) **Abstract**

To develop system technology for a high throughput in vitro synthesis reaction method for biopolymers such as proteins, RNA, and the like. In detail, a cell-free synthesis system wherein a template substance is used as a raw material, and an automatic synthesizer using the system, includes the following systems, control systems therefor, or combinations thereof;
1) to make a template substance, a substrate, and a reaction solution into contact so as to lead to a synthetic reaction,
2) to put the reaction system outside of the synthetic reaction system to perform dilution treatment of the solution approximately around the decrease in the synthesis velocity, approximately around the stop of the synthetic reaction, or in the middle thereof,
3) to perform concentration treatment subsequent to the dilution treatment, and
4) to put the reaction system back into the synthetic reaction system,

or
1) to make a template substance, a substrate, and a reaction solution into contact so as to lead to a synthetic reaction,
2) to put the reaction system outside of the synthetic reaction system to perform concentration treatment of the solution approximately around the decrease in the synthesis velocity, approximately around the stop of the synthetic reaction, or in the middle thereof,
3) to perform dilution treatment subsequent to the concentration treatment, and
4) to put the diluted reaction system back into the synthetic reaction system.

## Description

Priority is claimed on Japanese Patent Application No. 2003-122930 and Japanese Patent Application No. 2003-281500, the contents of which are incorporated herein by reference.

### Technical Field

The present invention relates to a cell-free synthesis system using a template substance. More precisely, it relates to a system for maximizing the efficiency of a synthetic reaction when a substance is synthesized using a template substance as the raw material. Moreover, it relates to a synthesizer for automatically performing the system.

### Background Art

As a method of synthesizing a biopolymer such as a protein or RNA in vitro, a so-called batch method has been used from old times, in which substrates, ions, buffers, cell extract, and synthetases required for the reaction are prepared and reacted in vitro. However, attributable to the reaction principle, the batch reaction method has a large limitation in that, due to a decrease in the substrate concentration as the reaction is in progress and an increase in the concentration of by-products having a synthesis-inhibitory effect, the synthetic reaction is stopped within a short time, resulting in a small amount of the target product. A. Spirin et. al. in the former Union of Soviet Socialist Republics have published a continuous method of synthesizing cell-free protein wherein, by using column chromatography having an ultrafiltration membrane, a dialysis membrane, or a resin fixed with a translational template, an energy source such as an amino acid, ATP, or GTP and a substrate can be continuously supplied, and at the same time the target product and the by-products can be continuously removed from the reaction system (Non Patent Document 1). However, in the continuous method using a semipermeable membrane or an ultrafiltration column, a complex apparatus must be assembled and adequate experience is required since the processing is complicated. Moreover, at the same time, the method has a large drawback difficult point for automating the reaction system.

As a method to solve the above problems, Endo et. al. have invented a contact interface diffusion method wherein a substrate can be supplied and the by-products can be removed continuously without using a semipermeable membrane (Patent Document 1). This method has enabled synthesis of a large amount of proteins without using a special reactor. However, since the method is based on the molecular diffusion principle, a drawback is that it takes a long time to synthesize a required amount of proteins, and this problem is yet to be solved.

Furthermore, in the RNA synthesis method in vitro, particularly in the high throughput synthesis method of mRNA serving as the essential translational template for cell-free protein synthetic reaction, problems as seen in the method of synthesizing cell-free protein still remain to be solved. As a study of the RNA synthesis method, Endo et. al. have developed a continuous synthesis method using a dialysis membrane, and found that this method is an RNA synthesis method which results in extremely high synthetic yield, greatly exceeding the performance of the batch method (Patent Document 2). However, in the RNA continuous synthesis method using a semipermeable membrane or the like, a complex apparatus must be assembled, and since the processing is complicated, adequate experience is required. Moreover, the method has a large drawback for automating the reaction system.

The requirements for the high throughput synthesis method of a biopolymer such as a protein or RNA in today's post genomic era are considered as follows: 1) to be able to synthesize a large amount in a short time; 2) to be able to synthesize many types of molecules in a short time; 3) to be able to become an elemental technology for automating the reactor based on a simple principle, and so on. In particular, the requirements 1) and 2) are very important not only from the viewpoint of cost reduction of the synthesis, but also for preparation while maintaining the activity of a protein having relatively unstable physical properties.
(Patent Document 1) WO02/24939
(Patent Document 2) WO01/27260
(Non Patent Document 1) Spirin, A. et al. (1993) Meth. in Enzymol., 217, 123-142.

### Disclosure of the Invention

An object of the present invention is to provide system technology for a high throughput in vitro synthesis reaction method for biopolymers such as proteins and RNA, which is completely different from the principle of the continuous synthesis method using a complicated device such as a semipermeable membrane, and an automatic synthesizer using such a system. This enables simple and efficient comprehensive preparation and mass production for analyzing the structure and function of RNA, proteins and the like serving as a gene product.

In order to solve the above problem, various examinations were performed with an object of maintaining high reaction velocity for a long time in synthesis systems having a template substance as a raw material. As a result, the present invention achieved a solution of the problems by completing a cell-free system including the following systems, a control system therefor, or combinations thereof, and an automatic synthesizer using the system.

That is, the present invention is as follows.
1. A cell-free synthesis system wherein a template substance is used as a raw material, including the following systems, a control system therefor, or combinations thereof;
   1) to make a template substance, a substrate, and a reaction solution into contact so as to lead to a synthetic reaction,
   2) to perform dilution treatment of the reaction solution approximately around the decrease in the synthesis velocity, approximately around the stop of the synthetic reaction, or in the middle thereof,
   3) to perform concentration treatment subsequent to the dilution treatment, and
   4) to perform synthetic reaction by the concentrated reaction system,

   or
   1) to make a template substance, a substrate, and a reaction solution into contact so as to lead to a synthetic reaction,
   2) to perform concentration treatment of the reaction solution approximately around the decrease in the synthesis velocity, approximately around the stop of the synthetic reaction, or in the middle thereof,
   3) to perform dilution treatment subsequent to the concentration treatment, and
   4) to perform synthetic reaction by the diluted reaction system.
2. The system according to item 1 wherein by-products are removed from the reaction system in the concentration treatment.
3. The system according to item 1 or 2, wherein the substrate, an energy source, and/or the template substance is replenished into the reaction system in the dilution treatment.
4. The system according to item 1, wherein the systems of 1) to 4) are repeated for a plurality of times.
5. The system according to any one of items 1 to 4, wherein the template substance is a transcriptional template.
6. The system according to any one of items 1 to 4, wherein the template substance is a translational template.
7. The system according to item 6, wherein the reaction solution contains a cell extract.
8. A cell-free synthesis system wherein a transcription product production system obtained from the system of item 5, and a translation product production system obtained from the system of item 6 or 7 are combined.
9. A synthetic kit including at least one reagent used for the cell-free system synthetic method using a template substance, which uses the system according to any one of items 1 to 8.
10. A cell-free system synthetic method using a template substance, which uses the system according to any one of items 1 to 8.
11. A cell-free system synthesizer using a template substance, which uses the system according to any one of items 1 to 8.
12. A cell-free system synthesizer for automatically executing the system according to any one of items 1 to 8, comprising at least the following control systems for executing a plurality of times a synthesis system using the initial phase in a synthetic reaction having a high protein synthetic reaction velocity;
   (1) a system for starting the synthesis;
   (2) a system for concentrating the reaction liquid;
   (3) a system for diluting the reaction liquid;
   (4) a system for reactivating the synthetic reaction; and
   (5) a system for repeating the operations of the systems of (2) to (4) ;

   or
   (1) a system for starting the synthesis;
   (2) a system for diluting the reaction liquid;
   (3) a system for concentrating the reaction liquid;
   (4) a system for reactivating the synthetic reaction; and
   (5) a system for repeating the operations of the systems of (2) to (4).
13. The synthesizer according to item 12, wherein the system for concentrating the reaction liquid uses an ultrafiltration membrane and, when the reaction liquid is removed from the reaction system through the ultrafiltration membrane, substances in the reaction liquid which can not pass through the ultrafiltration membrane are concentrated in the reaction system.
14. The synthesizer according to item 13, wherein a centrifugal separator and/or a suction pump is used for removing the reaction liquid from the reaction system in the system for concentrating the reaction liquid.
15. The synthesizer according to item 12, wherein the system for diluting the reaction liquid is to add a diluting solution or a substrate solution into the reaction liquid.
16. The synthesizer according to any one of items 11 to 15, at least comprising, in a step for synthesizing a cell-free protein, one of the following control systems for automatically performing the step until the protein encoded by the template is produced from the transcriptional template;
   (1) a system which variably controls the temperature in the reaction container;
   (2) a system which dispenses a sample or a reagent into the reaction container;
   (3) a system which transfers the reaction container; and
   (4) a system for precipitation and concentration filtration.
17. A program for executing the system according to any one of items 1 to 8, including the following information processing means for executing a plurality of times a synthesis system using the initial phase in a synthetic reaction having a high protein synthetic reaction velocity;
   (1) an information processing means for setting the synthesis time within a range of an initial phase in the synthetic reaction before the synthetic amount per unit time tends to decrease, based on information on the volume of the reaction container and the concentration of the reaction liquid,
   (2) an information processing means for setting the reaction liquid out of a range of the concentration in which the synthetic reaction is substantially possible, by adding a diluting solution into the reaction container when the time comes up to the synthesis time within the range of the initial phase in the synthetic reaction of (1),
   (3) an information processing means for concentrating the reaction liquid in the reaction container after (2) to set so that the liquid volume that has been increased by adding the diluting solution, can be brought back to the initial liquid volume,
   (4) an information processing means for setting to an optimum temperature for reaction after (3) so as to restart the synthetic reaction, and
   (5) an information processing means for repeating (1) to (4) for a plurality of times,

   or,
   (1) an information processing means for setting the synthesis time within a range of an initial phase in the synthetic reaction before the synthetic amount per unit time tends to decrease, based on information on the volume of the reaction container and the concentration of the reaction liquid,
   (2) an information processing means for setting the reaction liquid out of a range of the concentration in which the synthetic reaction is substantially possible, by concentrating the reaction liquid in the reaction container when the time comes up to the synthesis time within the range of the initial phase in the synthetic reaction of (1),
   (3) an information processing means for setting so as to add the diluting solution into the reaction container after (2) so that the liquid volume that has been decreased by concentrating, can be brought back to the initial liquid volume,
   (4) an information processing means for setting to an optimum temperature for reaction after (3) so as to restart the synthetic reaction, and
   (5) an information processing means for repeating (1) to (4) for a plurality of times.
18. A cell-free system synthesizer installed with the program according to item 17, wherein by performing the information processing of the program jointly with the synthesizer; a means for starting the synthesis, a means for diluting/concentrating the reaction liquid, and a means for reactivating the synthetic reaction, are executed; and the synthesis system using the initial phase in the synthetic reaction having the high protein synthetic reaction velocity can be executed repeatedly for a plurality of times.
19. A protein synthesized by the synthesizer according to items 11 to 16 and 18.

### Brief Description of the Drawings

Fig. 1 is a graph showing the difference in experimental results between the method of synthesizing cell-free protein of the present invention and the conventional cell-free protein method by the batch method.
Fig. 2 is a graph showing experimental results of cell-free protein synthesis performed similarly to example 1 except for the difference in the concentration of wheat embryo extract.
Fig. 3A is a graph showing experimental results of cell-free protein synthesis performed similarly to example 1 except for the difference in that the concentration of the wheat embryo extract was set to 80 OD, the concentration of GFP mRNA was set to 640 µg/ml, and the time interval for discontinuously repeating the dilution and concentration was changed.
Fig. 3B is results of a polyacrylamide gel electrophoresis showing the difference in the synthesized GFP amount due to the difference in the interval for discontinuously repeating the dilution and concentration.
Fig. 4 is a diagram schematically showing an example of a configuration wherein a concentrator having a filtration membrane and a liquid sending pump are combined so as to remove by-product in the method of synthesizing cell-free protein of the present invention.
Fig. 5 is a graph showing experimental results of protein synthesis in the method of synthesizing cell-free protein of the present invention performed using the configuration shown in Fig. 4 where the filtration membrane and the liquid sending pump are used for removing by-products.
Fig. 6 A shows experimental results in a case where cell-free protein synthesis was performed similarly to example 1 using a translational reaction liquid similar to example 1 except for the difference in that the concentration of the wheat embryo extract was set to 60 OD, 450 µg/ml of mRNA of dihydrofolate reductase (DHFR) was used as the translational template, and the dilution and concentration was discontinuously repeated twice at intervals of 1 hour.
Fig. B is an electrophoretogram showing experimental results where GFP fusion protein having streptavidin fused on the N-terminus was synthesized by the method of the present invention, and the GFP fusion protein was isolated from the system.
Fig. 7 shows a difference in experimental results between the method of synthesizing RNA of the present invention and a conventional method of synthesizing RNA in vitro by the batch method.
Fig. 8 is a graph showing experimental results of the method of synthesizing cell-free protein of the present invention performed using mRNA purified by ethanol precipitation and unpurified mRNA respectively as the translational template.
Fig. 9 is a graph showing experimental results of the method of synthesizing cell-free protein of the present invention performed with a translational reaction liquid that had been prepared using a transcriptional reaction liquid as it was after a transcriptional reaction of experimental example 7.

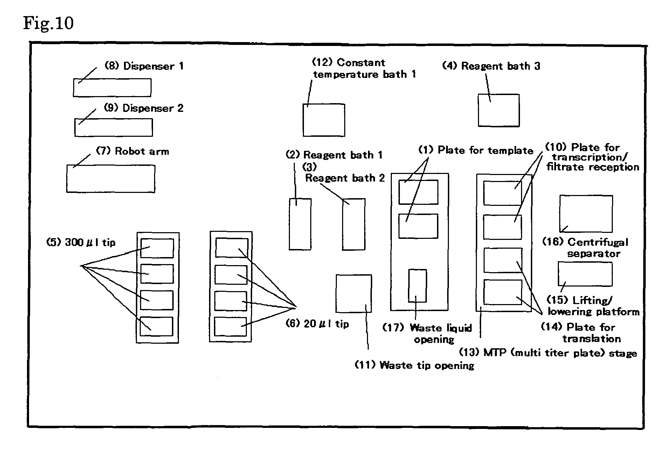

Fig. 10 is a schematic diagram of an automatic synthesizer.
Fig. 11 is an SDS-PAGE showing the results of examples of the apparatus.

### (Description of the Reference Symbols)

1: Filtration concentrator
2: Liquid sending pump
3: Reaction bath
4: Container containing (RNA) substrate solution
5: Liquid sending switch valve
6: Container
T1: Tube T1
T2: Tube T2
T3: Tube T3
T4: Tube T4
T5: Tube T5
(1): Plate for template
(2): Reagent bath 1 (solution for transcriptional reaction)
(3): Reagent bath 2 (solution for translational reaction)
(4): Reagent bath 3 (diluting solution)
(5): 300 µl tip
(6): 20 µl tip
(7): Robot arm
(8): Dispenser 1
(9): Dispenser 2
(10): Plate for transcription/filtrate reception
(11): Waste tip opening
(12): Constant temperature bath 1
(13): MTP (multi titer plate) stage
(14): Plate for translation
(15): Lifting/lowering platform
(16): Centrifugal separator
(17): Waste liquid opening

### Description of the Preferred Embodiments

The present invention is a synthesis method of a general batch type or a diffusion continuous batch type using a template substance as a raw material wherein properties of the initial phase in a synthetic reaction having a high reaction velocity are utilized to the maximum. Moreover, the present invention is a synthesizer for enabling automation of the synthesis method utilizing the properties to the maximum.

The means is to perform dilution treatment or concentration treatment of the reaction solution approximately around the decrease in the synthesis velocity, approximately around the stop of the synthetic reaction, or in the middle thereof. In the dilution treatment or the concentration treatment, components such as a substrate, an energy source, ions, buffers, and a template substance required for the synthesis using the template substance as a raw material, are replenished or concentrated.

The reaction solution which has been diluted or concentrated is then subjected to concentration treatment when it has been diluted, or dilution treatment when it has been concentrated. In the treatment, the reaction solution is brought back to the initial optimum concentration for reaction. In the concentration treatment, the components in the reaction solution are removed or separated, and the reaction product and/or the reaction by-products are collected and/or removed. By the concentration or the dilution, the respective components of the synthetic reaction are prepared to the optimum concentration again. The discontinuous repetition of the dilution treatment and the concentration treatment enables synthesizing a large amount of proteins.

The principle of the present invention is the synthesis method in which dilution and concentration are discontinuously repeated in the synthetic reaction by the template substance.

Moreover, the principle of the automatic synthesizer of the present invention is the automation of the synthesis method in which dilution and concentration are discontinuously repeated.

In the present invention, one of the synthesis systems using a template substance as a raw material is a protein translational synthesis system using mRNA as a template, in particular a cell-free protein synthesis system which uses the protein-synthesizing cell extract utilizing a ribosome or the like serving as an intracellular protein translator by taking out from a cell. Another synthesis system means an RNA synthesis system by an in-vitro transcriptional reaction from a transcriptional template using RNA polymerase as an enzyme.

### (Cell-free protein synthesis system)

Among cell-free protein synthesis systems, the system using a wheat embryo extract particularly shows a low nuclease activity, with the property of stable and high translational activity, compared to the cell-free protein synthesis system using an Escherichia coli extract (Madin K. et. al., (2000) Proc. Natl. Acad. Sci. USA 97, 559-564). Therefore, a transcriptional/translational system using a plasmid as a template enables synthesizing a protein with high efficiency (PCT/JP 99/04088). Hereunder, the present invention is described using the wheat embryo extract cell-free protein synthesis system and the method of synthesizing a transcriptional template which can function in the synthesis system as an example. However, the basic principle of the present invention is applicable to a cell-free protein synthesis system using another cell extract derived from a microorganism or an animal, or a synthesis system of a transcriptional template used for it.

### (Cell extract)

Examples of commercial products of the cell extract include E. coli S30 extract system (Promega) and RTS 500 Rapid translation System (Roche) derived from Escherichia coli, Rabbit Reticulocyte Lysate System (Promega) derived from rabbit reticulocyte, Proteios (™) (TOYOBO) derived from wheat embryo, and the like. In particular, a embryo extract derived from plant seed is preferably used, examples of which include seeds of wheat, barley, rice, corn, spinach, and the like. The most optimum is a system using a wheat embryo extract.

### (Translation product production system)

Here, the translation product production system is a method performed in vitro in which components containing a ribosome serving as an intracellular protein translator and the like are extracted from an organism, and the extract is then added with a translational template, a nucleic acid serving as a substrate, an amino acid, an energy source, various ions, buffers, and other effective factors.

### (Cell-free protein synthesis system)

Here, the cell-free protein synthesis system is a method performed in vitro in which components containing a ribosome serving as an intracellular protein translator and the like are extracted from an organism, and the extract is then added with a transcriptional or translational template, a nucleic acid serving as a substrate, an amino acid, an energy source, various ions, buffers, and other effective factors.

### (Wheat embryo extract)

For the cell extract-containing liquid of the present invention, a liquid containing a wheat embryo extract is suitable.

For the preparation method of the wheat embryo extract, for example the method described in Johnston, F. B. et al., Nature, 179, 160-161 (1957) can be used as a method of isolating the wheat embryo, and for example the method described in Erickson, A. H. et al., (1996) Meth. In Enzymol., 96, 38-50 can be used as a method of extracting a cell extract-containing liquid from the isolated embryo. Other examples include methods of Japanese Patent Application No. 2002-23139 and Japanese Patent Application No. 2002-231340.

In the embryo extract suitably used in the present invention, substances that suppress the protein-synthesizing function (substances such as tritin, thionine, and ribonuclease that act on mRNA, tRNA, translational protein factor, ribosome, or the like to suppress its function) contained in or held by the wheat itself serving as a raw material, are almost completely removed. That is, the endosperm where the inhibitors are localized is almost completely removed and purified. The degree of endosperm removal can be evaluated by monitoring the activity of tritin contaminated in the wheat embryo extract, that is the activity of deadenylating the ribosome. If the ribosome is not substantially adenylated, it can be judged that there is no endosperm-derived component contaminated in the embryo extract, that is the endosperm is almost completely removed and purified. The degree to which the ribosome is not substantially deadenylated means that the deadenylating rate of ribosome is less than 7 %, and preferably 1 % or less.

The embryo extract contains proteins that are derived from the cell extract-containing liquid or that are separately added according to need. The content thereof is not specifically limited. However, from the point of preservation stability in the freeze-dry state and ease of use, it is preferably 1 to 10 weight %, and more preferably 2.5 to 5 weight % in the overall composition before freeze-drying, and it is preferably 10 to 90 weight %, and more preferably 25 to 70 weight % in the overall freeze-dried composition after freeze-drying. The protein content described here can be calculated by measuring the absorbancy (260, 280, 320 nm).

### (Removal of microorganism)

In the cell extract-containing liquid, microorganisms, in particular spores such as filamentous bacteria (mold) and the like, might be mixed. These microorganisms are preferably removed. Since the propagation of microorganisms may be found particularly during a long term cell-free protein synthetic reaction (a day or more), it is important to prevent the propagation. Although the removal method is not specifically limited, a filtration sterilization filter is preferably used. The pore size of the filter is not specifically limited provided it enables removing of the microorganisms that might be possibly mixed and, ordinarily, is suitably from 0.1 to 1 micro meter, and more preferably from 0.2 to 0.5 micro meter.

Furthermore, by adding a step for removing low molecular weight synthesis inhibitors and/or a step for reducing the reductant concentration, in any stage of the steps for preparing the cell extract-containing liquid, then the cell extract-containing liquid can be made a liquid for performing cell-free protein synthesis having a special effect.

### (Method of removing low molecular weight synthesis inhibitors from cell extract-containing liquid)

The cell extract-containing liquid contains synthesis inhibitors of low molecular weight having a protein-synthesis inhibitory activity (hereunder, it may be called "low molecular weight synthesis inhibitors"), and by removing them a cell extract-containing liquid having a high protein-synthesizing activity can be obtained. Specifically, the low molecular weight synthesis inhibitors are fractioned and removed from the components in the cell extract-containing liquid by the difference in the molecular weight. The low molecular weight synthesis inhibitor can be fractioned as a molecule having a molecular weight equal to or less than the smallest factor required for the protein synthesis contained in the cell extract-containing liquid. Specifically, it can be fractioned and removed as a molecule having a molecular weight of 50,000 to 14,000 or less, and preferably 14,000 or less.

For the method of removing low molecular weight synthesis inhibitors from the cell extract-containing liquid, a normally-used and known method in itself is used. Specific examples include a method by dialyzing through a dialysis membrane, a gel filtration method, an ultrafiltration method, and the like. Among them, the method by dialysis is preferable from the point of easiness of supplying the substance to the dialysis inner liquid.

Examples of the dialysis membrane that is used for the removal operation of the low molecular weight synthesis inhibitors by dialysis, include membranes having a removable molecular weight of 50,000 to 12,000. Specifically, a regenerated cellulose film (made by Viskase Sales, Chicago) having a removable molecular weight of 12,000 to 14,000, a spectra/pore 6 (made by SPECTRUM LABOTRATORIES INC., CA, USA) having a removable molecular weight of 50,000, and the like are preferably used. An appropriate amount of cell extract-containing liquid and the like is put into such a dialysis membrane and dialyzed using a usual method. The time length for the dialysis is preferably from 30 minutes to 24 hours.

### (Stabilization of cell extract-containing liquid)

If insoluble components are generated in the cell extract-containing liquid when the low molecular weight synthesis inhibitors are being removed, inhibition of the generation thereof (hereunder, it may be called "stabilization of the cell extract-containing liquid") can increase the protein-synthesizing activity of the cell extract-containing liquid or the solution for translational reaction that is finally obtained. An example of a specific method of stabilizing the cell extract-containing liquid or the solution for translational reaction include a method in which, when the low molecular weight synthesis inhibitors are being removed, the cell extract-containing liquid or the solution for translational reaction is added with at least a high-energy phosphate compound, for example ATP or GTP (hereunder, it may be called a "stabilizing component"). For the high-energy phosphate compound, ATP is preferably used. Moreover, the stabilization is performed in a solution containing ATP and GTP, and more preferably in a solution containing ATP, GTP, and 20 types of amino acids.

These components may be preferably added with stabilizing components and incubated, then supplied to a step for removing the low molecular inhibitors. When the dialysis method is used for removing the low molecular weight synthesis inhibitors, the stabilizing component may be added into the dialysis outer liquid too, to perform the dialysis so as to remove the low molecular weight synthesis inhibitors. It is more preferable to add the stabilizing component into the dialysis outer liquid too, since the new stabilizing component is always supplied even if the stabilizing component is decomposed during the dialysis. This is also applicable to the case where a gel filtration method or an ultrafiltration method is used. A similar effect can be obtained by equilibrating the respective carriers by a filtration buffer containing a stabilizing component, then supplying the cell extract-containing liquid or the solution for translational reaction containing the stabilizing component, and performing the filtration while adding the buffer.

The dosage of the stabilizing component and the time length for the stabilizing treatment may be appropriately selected according to the type and the preparation method of the cell extract-containing liquid. An example of the selection method includes a method of; adding the stabilizing components experimentally having various amounts and types into the cell extract-containing liquid, and after an appropriate amount of time, performing a step for removing the low molecular inhibitors, then separating the obtained post-treatment cell extract-containing liquid into soluble components and insoluble components by a method such as centrifugation, then selecting from these the one having the least insoluble components. Furthermore, also preferable is a method of performing the cell-free protein synthesis using the obtained post-treatment cell extract-containing liquid, then selecting the one having a high protein-synthesizing activity. Moreover, when the cell extract-containing liquid and the dialysis method are used in the above selection methods, another example includes a method of adding appropriate stabilizing components into the dialysis outer liquid, and performing the dialysis for an appropriate amount of time using them, then performing selection according to the amount of the insoluble components in the obtained cell extract-containing liquid, the protein-synthesizing activity of the obtained cell extract-containing liquid, and the like.

An example of a condition for stabilizing the cell extract-containing liquid selected in this manner, includes a method in which, specifically, when the step for removing the low molecular weight synthesis inhibitors is performed by the dialysis method, adding 100 µM to 0.5 mM of ATP, 25 µM to 1 mM of GTP, and 25 µM to 5 mM of 20 types of amino acids respectively into the wheat embryo extract-containing liquid and the dialysis outer liquid, and performing the dialysis for 30 minutes to 1 hour or more. The temperature for performing the dialysis may be any provided that the protein-synthesizing activity of the cell extract-containing liquid is not lost and the dialysis can be performed. Specifically, the minimum temperature is temperatures at which the solution is not frozen, being normally -10°C , and preferably -5°C, and the maximum temperature is 40°C being the limit at which the solution used for the dialysis is not affected, and preferably 38°C.

Moreover, if the low molecular weight synthesis inhibitors are removed after preparation as the cell extract-containing liquid, it is not necessary to further add the stabilizing component into the cell extract-containing liquid.

### (Preparation of mRNA)

The preparation method of mRNA serving as the template substance is not limited provided that the mRNA encodes the target protein, and any publicly known method may be used. Of course, it is particularly preferable to use mRNA that has been prepared from DNA as the template substance by means of the present invention, and furthermore to continuously use it in a series of systems.

### (Preparation of solution for translational reaction)

In the synthesis of protein from mRNA, a solution (translational reaction solution) containing: amino acids (20 types of essential amino acids, or analog or variant amino acids according to the object are used) serving as the substrate; an energy source (ATP and/or GTP); various ions (calcium ion, magnesium ion, ammonium ion, and the like) as required; buffers (HEPES-KOH, Tris-Acetic acid, and the like); ATP regenerating system (combination of phosphoenolpyruvate and pyruvate kinase, combination of creatine phosphate and creatine kinase, and the like); nuclease inhibitor (ribonuclease inhibitor, nuclease inhibitor, and the like); tRNA; reductant (dithiothreitol and the like); polyethylene glycol; 3',5'-cAMP; folic acid; antibacterial agent (sodium azide, ampicillin, and the like); and other components suitable for the translational reaction is added into a protein synthesizing cell extract containing a ribosome at a concentration suitable for the cell-free protein synthesizing system, which is then added with mRNA of the translational template, so as to prepare the solution for translational reaction. The solution for translational reaction is prepared to a temperature suitable for the translational reaction, to perform the translational reaction, and then the target protein is synthesized.

### (Transcriptional reaction)

The transcriptional template may be prepared according to the method (WO01/27260, WO02/18586) of constructing a template molecule having wide applicability, that has been already reported by the present inventor, a preferred example of which includes a method wherein a desired structural gene is introduced into a plasmid vector pEU and the transcription is performed by SP6RNA polymerase treatment. However, of course, the preparation method of mRNA is not limited to this. The transcriptional template is mixed with a solution (also called a transcriptional reaction solution) containing the necessary components for the transcriptional reaction such as an RNA polymerase that matches a promoter in the transcriptional template, and substrates (4 types of ribonucleoside-triphosphate) for RNA synthesis, and the like, after which the transcriptional reaction is performed by incubating it at about 20 to 60°C, preferably about 30 to 42°C, for about 30 minutes to 16 hours, preferably about 2 to 5 hours.

### (Synthetic reaction)

In the cell-free synthesis system of the present invention, a system including the following means, a control means therefor, or a combination thereof is performed.

The essential means 1 is to make the template substance, the substrate, and the reaction solution into contact so as to lead to the synthetic reaction system. The abovementioned template substance, the substrate, and the reaction solution (the transcriptional reaction solution or the solution for translational reaction) are prepared to an optimum concentration and an optimum temperature so as to perform an optimum synthetic reaction. The synthetic reaction is normally performed for about 10 minutes to 2 hours, preferably about 20 minutes to 1 hour. The time length may be changed according to the respective systems, and the optimum time may be adjusted by experimental repetition. In the discontinuous repetitive synthesis method of the present invention, it is particularly preferable that the treatment time of one course is relatively short.

### (Dilution or concentration)

The essential means 2 is to dilute or concentrate the reaction system approximately around the decrease in the synthesis velocity, approximately around the stop of the synthetic reaction, or in the middle thereof, as the abovementioned reaction time. "Approximately around the decrease in the synthesis velocity" means a timing when the synthetic amount of mRNA or protein per unit time starts to show a tendency to decrease from the maximum amount as the time goes by, and is generally understood not in terms of a point but in terms of a line. "Approximately around the stop of the synthetic reaction" means a level at which the synthetic amount is decreased to be substantially undetectable, and this case is also understood not in terms of a point but in terms of a line. "In the middle thereof" means an interval from the time when the synthesis velocity starts to decrease until the synthetic reaction is stopped. In order to obtain more preferable synthetic efficiency, the dilution or concentration treatment is performed on the reaction system, approximately around the decrease in the synthesis velocity. The optimum time length is 10 minutes to 1 hour.

The dilution or concentration is performed on the reaction system as follows.

The dilution is performed by adding 1 to 20 volumes, preferably 2 to 10 volumes of an aqueous solution into the reaction system. The aqueous solution contains a template substance, a substrate, and a reaction solution according to need. In particular, a solution containing a template substance, a substrate, and an energy source is preferably used. The concentration of the respective components to be added may be selected so as to prepare an optimum concentration for synthesis after the subsequent concentration treatment. The dilution brings the solution into a state where the synthetic ability of the reaction system is significantly reduced. This state is called "discontinuous" in the present invention.

For the concentration, it is possible to use various publicly known methods wherein: when the reaction liquid is removed from the reaction system, substances (for example, synthesized protein, ribosome, and the like) in the reaction liquid which can not pass through may be concentrated in the reaction system. Preferable examples include filtration treatment using an ultrafiltration membrane, treatment by a centrifugal separator, gel filtration treatment, a suction pump, and a method of generating a differential pressure in the liquid phase or gaseous phase. In the treatment, by using a centrifugal operation or a molecular sieve while adjusting the passage diameter of the membrane, the reaction product and the reaction by-products are separated and removed. The molecular weight cut-off size of the membrane, the centrifugal speed, and the gel filtration conditions can be optimally adjusted according to the publicly known physical properties of the target product of the treatment. In the present invention, a membrane with a molecular weight cut-off at 10,000 to 100,000 Da is preferably used.

In this concentration treatment, the reaction solution is concentrated to 1/5 to 2/3 the volume of the initial volume, resulting in a large shift in the optimum concentration for synthesis of the respective synthetic factors. The concentration brings the reaction system into the state where the synthetic ability thereof is considerably reduced. This state is called "discontinuous" in the present invention.

### (Reactivation of synthetic reaction)

The essential means 3 is to reactivate the synthetic reaction. The diluted solution in the previous step is subjected to concentration treatment, and the concentrated solution in the previous step is subjected to dilution treatment.

In the former case, the dilution treatment is followed by the concentration treatment. Here, the "concentration" means to bring the liquid volume of the reaction system that has been increased by the dilution back, to the initial liquid volume. The concentration system is not specifically limited, and any publicly known concentration methods can be used. Preferable examples include filtration treatment using an ultrafiltration membrane, treatment by a centrifugal separator, gel filtration treatment, and a method of generating a differential pressure in the liquid phase or gaseous phase. In the treatment, using the centrifugal operation or a molecular sieve while adjusting the passage diameter of the membrane, the reaction product and the reaction by-products are separated and removed. The molecular weight cut-off size of the membrane, the centrifugal speed, and the gel filtration conditions can be optimally adjusted according to the publicly known physical properties of the target product of the treatment. In the present invention, a membrane with a molecular weight cut-off at 10,000 to 100,000 Da is preferably used.

In the latter case, the concentration treatment is followed by the dilution treatment. Here, the "dilution" means to bring the liquid volume of the reaction system that has been decreased by the concentration, back to the initial liquid volume. The diluting solution contains a template substance, a substrate, and a reaction solution according to need. In particular, a solution containing a template substance, a substrate, and an energy source is preferably used. The concentration of the respective components to be added may be selected so as to prepare an optimum concentration for synthesis after the dilution.

Thus, the reaction system restored to the optimum concentration of the reaction system is re-adjusted to the optimum temperature for reaction, so as to reactivate the reaction system. The optimum temperature is 15 to 25°C.

In order to separate, collect, and remove the reaction product and/or the reaction by-products, a treatment based on the affinity with the treatment object may be also preferably performed. An example of "based on the affinity" includes a method in which an affinitive substance is immobilized and made to contact with the target substance to bond them, and then the target substance is eluted and collected. Examples of affinitive substance includes: an antibody to a protein when the collection substance is a protein for example; a ligand to a receptor; and a nucleic acid to a transcription factor. A method is also possible in which the target product is modified with an appropriate tag marker (for example, streptavidin, histidine tag, GST, maltose binding protein, or the like), then purified using a substance (for example, biotin, bivalent metal ion, glutathione, maltose, or the like) which is bindable specifically with the modification substance.

In the present invention, the dilution and concentration treatment can be discontinuously repeated for a plurality of times. Such repetition achieves the regeneration of the cell-free synthesis system for a plurality of times. The regeneration achieves the synthesis of a large number of proteins.

In the present invention, the dilution and concentration treatment can be performed in combination with the control system as a series of steps. For the control, ON/OFF of the operation, the degree of the operation, the operation speed, and the interval of the operations are controlled by a drive source (a motor, a pneumatic/hydraulic device, and other operation-controllable actuators), a computer- controlled control circuit, a sequence control circuit or the like. Moreover, a driver for signal transfer, a sensor for operation check, a switch for operation control, a timer, or the like can be furnished according to need.

As described above, the present invention achieves the regeneration of the cell-free synthesis system by discontinuously repeating the dilution and concentration for a plurality of times. Hereunder is a more specific description of the effect.

### (Dilution-concentration batch type method of synthesizing cell-free protein)

In the present synthesis method, regarding the synthetic reaction container, it is possible to start the synthetic reaction using a container that has been conventionally used fitted with an ultrafiltration membrane or a dialysis membrane to enable concentration, as a batch reaction container. Using the cell-free protein synthesizing method, under optimum conditions, after the protein synthetic reaction, for example around the stop of the synthetic reaction, in order to reactivate the reaction system, a solution (being a dialysis outer liquid described in Madin K. et al., Proc. Natl. Acad. Sci. USA (2000), 97, 559-556, and abbreviated to a substrate solution hereunder) containing components such as a substrate, an amino acid, ATP and GTP serving as an energy source, buffers, and ions required for the protein synthesis and mRNA of the template substance is added into the reaction liquid at once to 1 to 20 volumes for dilution (dilution treatment). Then the reaction container is for example centrifuged by a centrifugal separator so that the additional solution in the container is discharged through the filtration membrane and the reaction liquid is brought back to the initial volume (concentration treatment). The concentration treatment can be performed not only by centrifugal force but also by generating a differential pressure in the liquid phase or gaseous phase. By the dilution and concentration treatment, fresh substrate solution can be supplied to the reaction system and by-products inhibiting the translational reaction that have been generated in the synthetic reaction can be removed at the same time. In this manner, by discontinuously repeating the dilution and concentration of the reaction liquid using a solution containing substrates and the like around the stop of the synthetic reaction for example, the reactivation of the reaction system can be achieved and the protein can be synthesized over a long period of time. The method is different in principle from the so-called continuation method developed by Spirin et. al. in which a semipermeable membrane is used to continuously supply the substrate and the energy source and dispose metabolites, and the effect of the synthesis reaches from ten to a thousand times (the method of the present invention enables obtaining the same output in 2 to 3 hours which has been obtained in about 30 hours in the method using a semipermeable membrane). Thus, the present method is largely different from the continuation method in terms of the quality. In the protein synthetic system using the present principle, selection of the molecular weight cut-off size of the filtration membrane to be used makes it possible to remove by-products and to fractionate and isolate the synthesized protein selectively from the reaction system at the same time. That is, use of the present principle makes it possible to synthesize the protein and to purify the synthesized product at the same time. Similar effects can be also achieved in the case of changing the order such that the dilution is performed subsequent to the concentration.

### (Dilution-concentration batch in vitro RNA synthesis method: Transcription product production system)

The general composition of the RNA synthetic reaction liquid using a phage RNA synthetase, reaction method, and method of analyzing the synthesized RNA followed the methods described in WO01/27260 and WO02/18586. In the synthesis method of the present invention, it is possible to start the synthetic reaction using a conventional container with an ultrafiltration membrane or a dialysis membrane to enable concentration, as a batch reaction container. The size of the molecular weight cut-off size of the ultrafiltration membrane to be used, is selected so that the RNA can not pass through. Around the stop of the synthetic reaction, a solution (which is a dialysis outer liquid prepared based on the method described in WO01/27260 and WO02/18586, and abbreviated to an RNA substrate solution hereunder) containing components such as 4 types of ribonucleotide-triphosphate serving as a substrate, ions, and buffers required for the RNA synthesis is added into the reaction liquid to 1 to 20 volumes for dilution. The reaction container is centrifuged by a centrifugal separator so that the additional solution in the container is discharged through the filtration membrane and the reaction liquid is concentrated to the initial volume. The treatment in this process can be performed not only by centrifugal force but also by generating a differential pressure in the liquid phase or gaseous phase. By these treatments, a fresh substrate solution is supplied to the reaction system, and mononucleotide and pyrophosphoric acid that have been generated in the batch RNA synthetic reaction are removed from the system at the same time. As a result, the RNA synthetic reaction that has been substantially stopped due to the principle of chemical equilibrium, is restarted by shifting the equilibrium. By repeating the dilution and concentration of the reaction liquid using substrates around the stop of the synthetic reaction, RNA can be synthesized over a long period of time. The method is different in principle from the RNA continuous synthesis method (WO00/68412) using dialysis previously invented by Endo et. al. In the protein synthetic system using the present principle, by repeating the dilution using water (or, desired solution) and concentration through the filtration membrane or the like on the reaction liquid after the synthesis, RNA not containing nucleotides, buffer components, or ions can be collected in water (or, as a solution containing target components). That is, in this method, by selecting the DNA molecular species serving as the template, a desired mRNA can be prepared as a solution at high speed, high yield, and low cost (since the expensive RNA synthetase is only used for the amount required for the normal batch system).

Similar effects can be also achieved in the case of changing the order such that the dilution is performed subsequent to the concentration.

### (Automatic synthesizer)

The automatic synthesizer of the present invention provides a method of automatically performing a series of reaction operations in the synthesis method of discontinuously repeating the dilution and the concentration with respect to at least a synthetic reaction (protein synthesis) by a translational template. Furthermore, it provides a method of automatically performing the reaction operation from the transcriptional template synthesized in the transcription product production system until the protein encoded by the template is generated. Here, "automatically performing the reaction operation" means that an experimenter does not directly and manually operate the reaction system (reaction container) during a series of steps. Consequently, when the respective steps are being performed, even if the experimenter manually operates the predetermined operation buttons or switches provided for the automatic synthesizer of the present invention to be used, the requirements of "automatic" in the present invention are not influenced.

In the present invention, the apparatus for automatically performing the reaction operation from the transcriptional template synthesized in the transcription product production system until the protein encoded by the template is generated; comprises at least the following systems (1) to (5).

Hereunder is a detailed description of the respective steps with specific embodiments. However, the method of the present invention is not intended to be limited to these provided it has the characteristics of a step for purifying the translational template.

### (1) Step for producing transcriptional template

In the automatic synthesizer of the present invention, the present step is not necessarily automatically performed, and a manually obtained transcriptional template may be used for the following automation step. However, it is more preferable to automatically perform a series of steps, from the production of the transcriptional template to the generation of the protein encoded by the template, including the present step.

In the present specification, the "transcriptional template" means a DNA that can be used as a template molecule of an in vitro transcriptional reaction, having at least a base sequence encoding the target protein on the downstream of an appropriate promoter sequence. The "appropriate promoter sequence" means a promoter sequence which can be recognized by an RNA polymerase used in the transcriptional reaction, for example, including an SP6 promoter, a T7 promoter, and the like. The DNA that encodes the target protein may be any type.

The transcriptional template preferably has a base sequence, which has an activity for controlling the translational efficiency, between the promoter sequence and the base sequence encoding the target protein. For example, a 5' untranslated region derived from an RNA virus such as an Ω sequence derived from a tobacco mosaic virus and/or a Kozak's sequence may be used. Furthermore, the transcriptional template preferably includes a 3' untranslated region including a transcription termination region on the downstream of the base sequence encoding the target protein. For the 3' untranslated region, about 1.0 to 3.0 kilobases on the downstream from the stop codon can be preferably used. The 3' untranslated region is not always necessarily originated from a gene that encodes the target protein.

The transcriptional template can be produced by a publicly known method. An example includes a method wherein: a host cell such as Escherichia coli having a plasmid inserted with a DNA including a base sequence identical to that of the desired transcriptional template is cultured; a large amount of the plasmid is prepared using a well-known purification method; then, the transcriptional template DNA is excised from the plasmid using an appropriate restriction enzyme; the restriction enzyme is removed by phenol treatment and chloroform treatment; and furthermore the transcriptional template is purified by alcohol precipitation using ethanol or isopropanol (an appropriate amount of salt such as ammonium acetate, sodium acetate, and sodium chloride is added according to need) or the like. The obtained DNA precipitation can be dissolved into extra pure water or the transcriptional reaction solution described later, and supplied to the following transcriptional reaction.

There is known an apparatus for automatically or semiautomatically (a state is intended where an experimenter directly and manually operates the reaction system in a part of the steps) performing such a series of operations. By incorporating such an apparatus into the automatic synthesizer of the present invention, it becomes possible to automatically perform the steps, from the production of the transcriptional template to the generation of the target protein. However, in consideration of the object of the present invention to provide a high throughput synthesis system according to the present invention for high throughput analysis, simplification of the apparatus, shortening of the required time, and the like, it is more preferable to use the method of producing the transcriptional template by a polymerase chain reaction (PCR) method as follows.

In the preferred embodiment of the synthesizer of the present invention, there is used a method in which the transcriptional template is amplified by directly applying a PCR to a host having a cloned DNA that encodes the target protein (for example, Escherichia coli having a plasmid including the DNA). For example, an oligonucleotide including: an appropriate promoter sequence; a 5' untranslated sequence having an activity for controlling the translational efficiency; and a part of a DNA 5' terminus region encoding the target protein, is synthesized using a DNA automatic synthesizer by a publicly known technique. This is used as a sense primer, and an oligonucleotide having a sequence of a 3' terminus region of a 3' untranslated sequence is used as an antisense primer. A host such as Escherichia coli having a plasmid including DNA that encodes the target protein as a template and the 3' untranslated sequence on the downstream thereof is directly added into the PCR reaction liquid, and then the amplification reaction is performed under normal conditions. As a result the desired transcriptional template can be obtained. In order to prevent the generation of short chain DNA (resulting in a decrease in the yield of the target product and noise of the low molecular translation product) caused by nonspecific amplification, the promoter scissioning primer described in International Publication No. 02/18586 pamphlet can be also used.

The amplification reaction can be performed in a commercial PCR 96-well plate using a commercial PCR thermal cycler, a similar temperature variable controller can be linked with the synthesizer of the present invention, or the respective systems for performing the transcriptional/translational reaction in the synthesizer of the present invention as they are can be applied directly-to the PCR.

The transcriptional template DNA obtained in the above manner may be supplied to the transcriptional reaction after purification by chloroform extraction or alcohol precipitation. However, in order to simplify the apparatus and shorten the required time, it is preferable to directly use the PCR reaction liquid as the transcriptional template solution. In the production of the transcriptional template, by using the PCR directly from the abovementioned host, the number of steps can be remarkably reduced and a large amount of the transcriptional template can be synthesized in a short time with less number of steps, compared to the case where a large amount of plasmid is once prepared, and then treated with restriction enzyme so as to obtain the transcriptional template. That is, by not requiring a step for preparing a large amount of plasmid by culturing Escherichia coli having a plasmid including the target gene, it becomes possible to shorten the time required for culturing, or for ultracentrifugation for purifying the plasmid. Moreover, since: the treatment with a restriction enzyme for excising the transcriptional template from the plasmid; the phenol treatment or chloroform treatment for removing the restriction enzyme and the like; the alcohol precipitation for purifying the transcriptional template; and the step for dissolving DNA serving as the transcriptional template can be omitted, then there is no inhibition against the transcriptional reaction due to remaining phenol/chloroform, nor loss in the transcriptional template due to the many steps of the purification operation. Moreover, since the number of steps required for the reaction can be reduced, there is another advantage of less number of tips to be used.

### (2) Step for transcriptional reaction

The synthesizer of the present invention includes a step for producing mRNA serving as the translational template from the transcriptional template DNA encoding the target protein that has been prepared using a publicly known method, by an in vitro transcriptional reaction. The present step is performed by mixing: a solution, preferably the above-described PCR reaction liquid, containing the transcriptional template that has been provided for the reaction system (for example, a commercial reaction container such as a 96 well-titer plate), with a solution (also called a "transcriptional reaction solution") containing necessary components for the transcriptional reaction such as an RNA polymerase (such as SP6 RNA polymerase) that matches a promoter in the transcriptional template, and substrates (4 types of ribonucleoside-triphosphate) for RNA synthesis and the like; after which the mixed liquid is incubated at about 20 to about 60°C, preferably about 30 to about 42°C, for about 30 minutes to about 16 hours, preferably about 2 to about 5 hours. The operations such as dispensing the transcriptional template solution and the transcriptional reaction solution into the reaction container, and mixing them can be performed by using a dispensing device of an automatic synthesizer described later (for example, a pipetter (if a commercial 96 well-titer plate is used as a reaction container, a device having 8- or 12-dispensing tips that matches with the well intervals is preferably used) or the like). Moreover, the incubation for the transcriptional reaction may be performed while controlling at a constant temperature by a temperature controlling system of the synthesizer described later.

### (3) Step for purifying translational template

The transcription product produced in the above manner (that is a "translational template") is an RNA molecule having a base sequence encoding the target protein, including a 5' untranslated sequence and/or a 3' untranslated sequence having an activity for controlling the translational efficiency inserted into the transcriptional template according to need. In the reaction liquid after the transcriptional reaction, unreacted ribonucleoside-triphosphate, pyrophosphoric acid being a reaction by-product, and salts contained in the solution for transcriptional reaction are mixed in addition to the translational template RNA. Since such substances are known to inhibit the translational reaction thereafter, they are removed by exchanging the reaction solution. An example of such a means for exchanging the solution includes a method in which the reaction solution is put into a container with a filter, and a solution containing substances inhibiting the translational reaction is removed by centrifugation. Then a solution such as an appropriate buffer is newly added, and this process is repeated. However, the method is not limited to this example. When the solution to be newly added is made to be the solution for translational reaction, the step can proceeds to the next translational reaction without purifying the template. Moreover, an example of a means for removing the substances includes a method in which the translational template is selectively precipitated so as to separate and remove the unreacted substrates and the like. An example of such a precipitation means includes salting out, and preferably an alcohol precipitation method. However, the means is not limited to these. When an alcohol precipitation method is used, the alcohol to be used is not specifically limited provided it can selectively precipitate RNA. However ethanol, isopropanol, and the like are preferable, and ethanol is more preferable. About 2 to 3 volumes of ethanol with respect to the transcriptional reaction liquid, and about 0.6 to 1 volume of isopropanol with respect to the transcriptional reaction liquid are preferably used. Moreover, by adding an appropriate concomitant salt, the yield of precipitation can be increased. Examples of such salt include ammonium acetate, sodium acetate, sodium chloride, and lithium chloride. For example, when ammonium acetate is used, it is desirable to add it so that the final concentration becomes about 0.5 M to about 3 M. Moreover, the alcohol precipitation may be performed at room temperature.

### (4) Step for translational reaction

The translational reaction can be performed by incubating the solution for translational reaction containing the translational template obtained in the above manner at a suitable temperature for the translational reaction, for an appropriate time. The translational reaction is normally performed for about 10 minutes to 2 hours, more preferably 20 minutes to 1 hour. The time length may be changed according to the respective systems, and the optimum time may be adjusted by experimental repetition. In the discontinuous repetitive synthesis method of the present invention, it is particularly preferable that the treatment time of one course is relatively short.

As described above, in the reaction, the reaction system is diluted or concentrated approximately around the decrease in the synthesis velocity, approximately around the stop of the synthetic reaction, or in the middle thereof. "Approximately around the decrease in the synthesis velocity" means a timing when the synthetic amount of protein per unit time starts to show a tendency to decrease from the maximum amount as the time goes by, and is generally understood not in terms of a point but in terms of a line. "Approximately around the stop of the synthetic reaction" means a level at which the synthetic amount is decreased to be substantially undetectable, and this case is also understood not in terms of a point but in terms of a line. "In the middle thereof" means an interval from the time when the synthesis velocity starts to decrease until the synthetic reaction is stopped. In order to obtain more preferable synthetic efficiency, the dilution or concentration treatment is performed on the reaction system, approximately around the decrease in the synthesis velocity. The optimum time length is 10 minutes to 1 hour.

The dilution or concentration is performed on the reaction system as follows.

The dilution is performed by adding 1 to 20 volumes, preferably 2 to 10 volumes of an aqueous solution into the reaction system. The aqueous solution contains a template substance, a substrate, and a reaction solution according to need. In particular, a solution containing a template substance, a substrate, an energy source and the like is preferably used. The concentration of the respective components to be added may be selected so as to prepare an optimum concentration for synthesis after the subsequent concentration treatment. The dilution brings the solution into a state where the synthetic ability of the reaction system is significantly reduced. This state is called "discontinuous" in the present invention.

For the concentration, it is possible to use any publicly known methods wherein: when the reaction liquid is removed from the reaction system, substances (for example, synthesized protein, ribosome, and the like) in the reaction liquid which can not pass through are concentrated in the reaction system. Preferable examples include filtration treatment using an ultrafiltration membrane, treatment by a centrifugal separator, gel filtration treatment, a suction pump, and a method of generating a differential pressure in the liquid phase or gaseous phase. In the treatment, by adjusting the passage diameter of the membrane and using a centrifugal operation or a molecular sieve, the reaction product and the reaction by-products are separated and removed. The molecular weight cut-off size of the membrane, the centrifugal speed, and the gel filtration conditions can be optimally adjusted according to the publicly known physical properties of the target product of the treatment. In the present invention, a membrane with a molecular weight cut-off at 10,000 to 100,000 Da is preferably used.

In this concentration treatment, the reaction solution is concentrated to 1/5 to 2/3 the volume of the initial volume, resulting in a large shift in the optimum concentration for synthesis of the respective synthetic factors. The concentration brings the solution into the state where the synthetic ability of the reaction system is considerably reduced. This state is called "discontinuous" in the present invention.

Subsequently, the synthetic reaction is reactivated. The diluted solution in the previous step is subjected to concentration treatment, and the concentrated solution is subjected to dilution treatment.

In the former case, the dilution treatment is followed by the concentration treatment. Here, concentration means to bring the liquid volume of the reaction system that has been increased by the dilution, back to the initial liquid volume. The concentration means is not specifically limited, and any publicly known concentration means can be used. Preferable examples include filtration treatment using an ultrafiltration membrane, treatment by a centrifugal separator, gel filtration treatment, and a method of generating a differential pressure in the liquid phase or gaseous phase. In the treatment, by adjusting the passage diameter of the membrane and using the centrifugal operation or a molecular sieve, the reaction product and the reaction by-products are separated and removed. The molecular weight cut-off size of the membrane, the centrifugal speed, and the gel filtration conditions can be optimally adjusted according to the publicly known physical properties of the target product of the treatment. In the present invention, a membrane with molecular weight cut-off at 10,000 to 100,000 Da is preferably used.

In the latter case, the concentration treatment is followed by the dilution treatment. Here, the dilution means to bring the liquid volume of the reaction system that has been decreased by the concentration, back to the initial liquid volume. The diluting solution contains a template substance, a substrate, and a reaction solution according to need. In particular, a solution containing a template substance, a substrate, and an energy source and the like is preferably used. The concentration of the respective components to be added may be selected so as to prepare an optimum concentration for synthesis after the dilution.

Thus, the reaction system restored to the optimum concentration of the reaction system is re-adjusted to the optimum temperature for reaction, so as to reactivate the reaction system. The optimum temperature is 15 to 25°C.

In the present invention, the dilution and concentration treatment can be discontinuously repeated for a plurality of times. Such repetition achieves regeneration of the cell-free synthesis system for a plurality of times. The repetition time is 2 to 20 times, and preferably 5 to 10 times. The regeneration achieves synthesis of a large number of proteins.

Other steps may be performed according to publicly known arbitrary procedures or conditions that can be applied for automation, and are not specifically limited.

In the present invention, an apparatus for performing the above operations comprises at least the following systems (a) to (e):
(a) a system which variably controls the temperature in the reaction container;
(b) a system which dispenses a sample or a reagent into the reaction container;
(c) a system which transfers the reaction container;
(d) a system for precipitation and concentration filtration;
   and
(e) a control system which controls to operate the systems of (a) to (d) according to the aforementioned method of the present invention.

By using an apparatus having at least the configuration of (a) to (e), it becomes possible to automatically perform the reaction operation, from the transcriptional template synthesized in the abovementioned transcription product production system in the present invention until the protein encoded by the template is generated. Hereunder is a detailed description of the respective configurations.

### (a) System which variably controls the temperature in the reaction container

A system which variably controls the temperature in the reaction container is a means for adjusting the temperature of a liquid in the reaction container to an appropriate temperature condition: in the transcriptional reaction; the incubation of the translational reaction; the stopping of the translational reaction; the precipitation of the translational template; or in the amplification reaction in the PCR method when the step for producing the transcriptional template by the PCR method is automatically performed using the automatic synthesizer of the present invention. The temperature range to be variably controlled is not specifically limited. The means is not specifically limited provided it is a device that can variably control the temperature of the liquid in the reaction container within a temperature range (for example, about 4°C to about 100°C, preferably about 15°C to about 60°C) that is normally required in a series of reaction operations of the cell-free protein synthesis including the production of the transcriptional template. Examples include conventionally publicly known TAKARA PCR Thermal Cycler MP (made by TAKARA BIO INC.), Gene Amp PCR System 9700 (made by Applied Biosystems Inc.), and the like. Specifically, this is achieved by providing a plurality of stages for mounting the reaction container, in the apparatus, separate from an operation stage for mounting the reaction container that is at a place for pipetting and so on, and variably controlling the temperature in the overall space above the stages so that the temperature in the reaction container can be variably controlled.

### (b) System which dispenses a sample or a reagent into the reaction container

A system which dispenses a sample or a reagent into the reaction container is a means for dispensing a sample or a reagent into the reaction container so as to perform a series of the cell-free protein synthetic reactions such as the transcriptional reaction, the translational reaction, and the PCR in the reaction container. Here, the "sample" denotes a transcriptional template, a translational template, a template plasmid for the PCR (or a host having a plasmid (for example, Escherichia coli)), and the like. The "reagent" denotes solution for a transcriptional reaction, a solution for translational reaction, a translational reaction solution, a diluting solution, an alcohol, a salt solution, a solution for PCR reaction, and the like. Such a dispensing system can be achieved by using a conventionally publicly known appropriate pipet arm (dispenser) capable of automatically dispensing and the like, without specific limitations, provided it can adjust the dosage of a sample or a reagent according to the step for dispensing. Moreover, the pipet arm may have a function of disposing used tips into a waste tip opening in the synthesizer, and a function of discharging the drawn filtrate and the like into a waste liquid opening.

Moreover, in addition to the above functions, the dispensing system preferably has a mixing function (for example, pipetting, stirring, and the like) for homogenizing the two or more types of solutions and dissolving the precipitation.

Furthermore, by adding the substrate solution or the diluting solution into the respective cells of the reaction container using the pipet arm, the dilution treatment of the reaction liquid can be performed.

### (c) System which transfers the reaction container

A system which transfers the reaction container is a means for moving the reaction container to the respective stages, the centrifugal separator, the lifting/lowering platform, and the constant temperature bath. Such a system which transfers the reaction container can be achieved by using a conventionally publicly known appropriate means without specific limitations, provided it can transfer the reaction container to the target destination. For example, it can be achieved by using a robot arm that is used for a conventional synthesizer.

### (d) System for precipitation and concentration filtration

A system for precipitation and concentration filtration is a means which precipitates opaque matter generated during the transcriptional reaction, or which concentrates and filtrates the reaction liquid at the time of the repeated translational reaction. Such a system can be achieved by using a conventionally publicly known appropriate means without any limitations, provided it can precipitate opaque matter generated during the reaction enabling the solid-liquid separation, and it can concentrate the reaction liquid at the time of the repeated transcriptional reaction. The system can be achieved by using for example, a conventionally publicly known centrifugal separator, or other appropriate device that is conventionally used for filtration or freeze drying.

### (e) Control system

A control system includes a controller which controls ON/OFF of the operation, the degree and the condition of the operation, and the like, of a drive source (a motor, a pneumatic/hydraulic device, and other operation-controllable actuators) used for the above systems (a) to (d) so that the respective systems can operate. The configuration of the control is so that the operation of the above systems (a) to (d), automatically performs the reaction operation, from the transcriptional template synthesized in the transcription product production system until the protein encoded by the template is generated.

The controller may be constituted by combining control equipment required to control the operation of the respective systems, such as a control circuit including a computer having a control program, a sequence control circuit, and the like. The control configuration is such that signals, and according to need, electric power, pneumatic pressure, hydraulic pressure, and the like can be supplied to the respective systems, so that the respective systems can operate in sequence according to the object. Moreover, a driver required for directly sending drive signals to the drive source of the respective systems, various sensors required for detecting the operation condition of the drive source of the respective systems, a switch, and the like may be appropriately added.

The reaction container that can be applied to the synthesizer of the present invention is not specifically limited, and it is possible to use various conventionally publicly known reaction containers that are used for cell-free protein synthetic reactions. Examples include a PCR 96-well plate, a 96 well-titer plate, 8-tubes, and a tube (1.5 ml, 15 ml, 50 ml, and the like). For example, when a batch method or a double layer method is used as a translational reaction system, the translational reaction can be performed in a small reaction system such as a 96-well plate. Moreover, according to the synthesizer of the present invention, since the transcriptional reaction can be also performed in a small reaction system, a series of reaction operations of the cell-free protein synthesis method including the transcriptional reaction, the purification of translational template, the translational reaction and, further according to need, the PCR for the production of the transcriptional template supplied to the translational reaction, can be performed in a plurality of reaction systems with respect to a plurality of types of proteins at once, so that a large amount of proteins can be synthesized in a short time.

Furthermore, an apparatus for performing the synthesis method in which dilution and concentration are discontinuously repeated in the synthetic reaction by the translational template is provided. The apparatus of the present invention comprises at least the following systems (1) to (5):
(1) a system for starting the synthesis;
(2) a system for concentrating the reaction liquid;
(3) a system for diluting the reaction liquid;
(4) a system for reactivating the synthetic reaction; and
(5) a system for repeating the system operations of (2) to (4);

or
(1) a system for starting the synthesis;
(2) a system for diluting the reaction liquid;
(3) a system for concentrating the reaction liquid;
(4) a system for reactivating the synthetic reaction; and
(5) a system for repeating the system operations of (2) to (4).

By using an apparatus having at least the configuration of (1) to (5), it becomes possible to automatically perform the abovementioned high throughput synthesis system of the present invention. Hereunder is a detailed description of the respective systems.

### (1) System for starting the synthesis;

(1) The dispenser is moved to a reagent bath containing the translational reaction liquid, and the translational reaction liquid is drawn.
(2) The dispenser is moved onto a plate for translation (the bottom of the cells is a filter), and the translational reaction liquid is discharged into the respective cells in the plate for translation containing the transcription product.
(3) Following from (2), the dispenser performs pipetting of the respective cells.
(4) The robot arm puts a lid on the plate for translation, transfers the plate to the constant temperature bath, and sets it in the constant temperature bath.
(5) The plate for translation is kept warm and the synthesis is started.
(6) After completion of a set synthesis time, the robot arm transfers the plate for translation onto the MTP stage.

### (2) System for diluting the reaction liquid;

(1) The dispenser is moved to a reagent bath containing the diluting solution, and the diluting solution is drawn.
(2) The dispenser is moved onto a plate for translation, and the diluting solution is discharged into the respective cells in the plate for translation containing the transcription product.

### (3) System for concentrating the reaction liquid

(1) The robot arm lays the plate for translation over the plate for transcription/filtrate reception.
(2) The robot arm transfers the laid plate for translation and the plate for transcription/filtrate reception onto the lifting/lowering platform.
(3) The lifting/lowering platform on which the laid plate for translation and the plate for transcription/filtrate reception are mounted, is lowered so as to match the height of the centrifugal separator.
(4) The robot arm sets the laid plate for translation and the plate for transcription/filtrate reception into the centrifugal separator. At this time, when there is one set of the plate for translation and the plate for transcription/filtrate reception, a dummy plate is set on the diagonal line. Moreover, when there are two sets, the plates are mutually set on the diagonal line.
(5) The centrifugation by the centrifugal separator is started. At this time, since the bottom of the cells is a filter, the filtrate is removed into cells in the plate for transcription/filtrate reception, and the respective cells in the plate for translation are concentrated.
(6) The robot arm transfers the laid plate for translation and the plate for transcription/filtrate reception from the centrifugal separator to the lifting/lowering platform.
(7) The lifting/lowering platform on which the laid plate for translation and the plate for transcription/filtrate reception are mounted, is lifted.
(8) The robot arm transfers the laid plate for translation and the plate for transcription/filtrate reception onto the MTP stage, and separately sets the plate for translation and the plate for transcription/filtrate reception.

### (4) System for reactivating the synthetic reaction

When the dilution treatment is first performed in the previous step, the concentration treatment is subsequently performed. When the concentration treatment is first performed in the previous step, the dilution treatment is subsequently performed.
(1) The robot arm transfers the concentrated plate for translation or the diluted plate for translation to the constant temperature bath, and sets it in the constant temperature bath.
(2) The plate for translation is kept warm and the synthesis is started.

### (5) System for repeating the system operations of (2) to (4)

(1) After a completion of a set time, the temperature of the constant temperature bath is decreased so as to stop substantial synthesis.
(2) The robot arm transfers the plate for translation onto the MTP stage.
(3) The system operations described in (2) to (4) are repeated for a plurality of times.

Moreover, the abovementioned systems of the synthesizer are only an example of an apparatus for automatically performing the high throughput synthesis system, and it may further include a means for disposing waste liquid from the concentration treatment, and a means for disposing waste tips from the dispenser.

Moreover, in order to perform the synthesis system using the initial phase in the synthetic reaction which is high in the protein synthetic reaction velocity for a plurality of times, for performing the high throughput synthesis system, the program including the following information processing means is also the subject of the present invention.
(1) an information processing means for setting the synthesis time within a range of the initial phase in the synthetic reaction before the synthetic amount per unit time tends to decrease, based on the information on the volume of the reaction container and the concentration of the reaction liquid,
(2) an information processing means for setting the reaction liquid out of the range of the concentration in which the synthetic reaction is substantially possible, by adding the diluting solution into the reaction container when the time comes up to the synthesis time within the range of the initial phase in the synthetic reaction of (1),
(3) an information processing means for concentrating the reaction liquid in the reaction container after (2) to set so that the liquid volume in the reaction system that has been increased by adding the diluting solution can be brought back to the initial liquid volume,
(4) an information processing system for setting to the optimum temperature for the reaction after (3) so as to restart the synthetic reaction, and
(5) an information processing means for repeating (1) to (4) for a plurality of times,

or,
(1) an information processing means for setting the synthesis time within a range of the initial phase in the synthetic reaction before the synthetic amount per unit time tends to decrease, based on the information on the volume of the reaction container and the concentration of the reaction liquid,
(2) an information processing means for setting the reaction liquid out of the range of the concentration in which the synthetic reaction is substantially possible, by concentrating the reaction liquid in the reaction container when the time comes up to the synthesis time within the range of the initial phase in the synthetic reaction of (1),
(3) an information processing means for adding the diluting solution into the reaction container after (2) to set so that the liquid volume in the reaction system that has been decreased by concentrating can be brought back to the initial liquid volume,
(4) an information processing means for setting to the optimum temperature for the reaction after (3) so as to restart the synthetic reaction, and
(5) an information processing means for repeating (1) to (4) for a plurality of times.

Furthermore, in a conventionally publicly known synthesizer installed with the above program, the synthesizer is also the subject of the present invention wherein: by performing the information processing of the program jointly with the synthesizer; a system for starting the synthesis, a system for diluting/concentrating the reaction liquid, and a system for reactivating the synthetic reaction, are executed; and the synthesis system using the initial phase in the synthetic reaction having the high protein synthetic reaction velocity can be performed for a plurality of times.

Moreover, the transcriptional reaction and the translational reaction are preferably performed in a sealed reaction container. From this viewpoint, it is preferable such that a reaction container with a lid is used. Furthermore the apparatus preferably has a system which opens/closes the lid of the reaction container. An example of the lid includes a rubber lid which can seal each well, for example when a 96- well plate is used as the reaction container. Since it is preferable that the lid can be tightly fitted with the reaction container when the lid is closed, it is considered to use a lid with a certain degree of weight (for example, about 500 g), or to close the lid by clamping the lid and the reaction container with something like a clip. Moreover, the system which opens/closes the lid of the reaction container can be achieved by using for example, a conventionally publicly known mechanism that is a combination of a chucking mechanism/suction mechanism and a robot arm.

The synthesizer of the present invention may have a system which stocks the reaction reagent according to need, in addition to the above systems.

As mentioned above, the high throughput system and the apparatus which automatically performs the system of the present invention can simply and automatically synthesis a plurality of types of proteins at the same time. For example, it is useful since a plurality of transcriptional templates and translational templates that encode proteins of various mutations can be prepared, and a plurality of proteins of a plurality of mutations can be synthesized at the same time so as to supply them for analysis and the like without requiring detailed design of the mutations.

Moreover, the high throughput system and the apparatus which automatically performs the system of the present invention can be suitably supplied for usage in high throughput function analysis of various proteins. For example: a gene group which encodes proteins including a common domain (for example, a kinase domain and the like) stored as a result of a homology search is made the template; the proteins are synthesized at the same time using the apparatus of the present invention by the method of the present invention, while on the other hand a protein group (for example, transcription factor and the like) that can be the target of phosphorylation is similarly synthesized; then both are mixed in various combinations; and for example the uptake of the ³²P labeled ATP is used as an index; so that it is possible to identify what type of protein kinase can phosphorylate what type of protein.

Alternatively: a gene group which encodes proteins including a motif peculiar to the transcription factor (for example, a Zn finger, a leucin zipper, and the like) is made the template; the proteins are synthesized at the same time using the apparatus of the present invention by the method of the present invention; and the binding with a known cis element sequence, the heterodimer forming ability with other transcriptional regulatory factors, and further the binding ability with the transcriptional regulatory regions of a specific gene promoter are examined; so that information for clarifying the crosstalk over a pattern of the transcription factors can be obtained.

### Example

Hereunder is a detailed description of the present invention with examples. However, the present invention is not intended to be limited to the following embodiments.

### (Method of synthesizing cell-free protein)

### 1) Reaction from DNA to mRNA (transcriptional reaction)

A transcriptional reaction solution [final concentration: 80 mM HEPES-KOH pH 7.8, 16 mM magnesium acetate, 10 mM dithiothreitol, 2 mM spermidine, 2.5 mM 4NTPs (4 types of nucleotide-triphosphate), 0.8 U/µl RNase inhibitor, 0.1 µg/µl DNA [plasmid GFP (Green fluorescent protein)], and 1.6 U/µl SP6 RNA polymerase] was prepared and reacted at 37°C for 3 hours. After the reaction, it was centrifuged at 12,000 rpm, at 4°C for 1 hour. After the centrifugation, the supernatant was collected, which was then added with ethanol and ammonium acetate so that the final concentration became about 70% of ethanol and about 0.27 M of ammonium acetate. It was allowed to stand at 4°C for 10 minutes on ice, and centrifuged at 3,000 rpm, for 30 minutes (ethanol precipitation). After performing the ethanol precipitation for 3 times, it was adjusted with an appropriate amount of dialytic buffer (final concentration: 35 mM HEPES-KOH pH7.8, 3.1 mM magnesium acetate, 103 mM potassium acetate, 16 mM creatine, 1.2 mM ATP, 0.26 mM GTP, 2.5 mM DTT, 0.43 mM spermidine, 0.3 mM various amino acids) so that the RNA concentration became about 5 to 10 mg/ml.

Moreover, the mRNA synthesis by the discontinuous repetition of dilution and concentration according to the present invention was performed according to experimental example 7.

### 2) Preparation of translational reaction solution

The embryo extract was prepared using the translational reaction solution [final concentration: 35 mM HEPES-KOH pH 7.8, 103 mM potassium acetate (KOAc), 3.1 mM magnesium acetate (Mg(OAc)₂), 16 mM creatine phosphate, 1.2 mM ATP, 0.26 mM GTP, 2.5 mM dithiothreitol (DTT), 0.43 mM spermidine, 0.3 mM AAs (mixture of 20 types of L-amino acid), 1.03 mg/ml creatine kinase] so that the concentration (O.D. 260 nm) of the embryo extract became from 40 to 120 at the time of the translational reaction, and then used.

### 3) Translational reaction

The mRNA solution that had been prepared above was added to the abovementioned translational reaction solution containing the embryo extract according to the concentration at the time of the translational reaction of the embryo extract, then the reaction was performed. That is, the mRNA amount to be added was prepared on a proportion basis assuming that the mRNA. amount to be added was set to 0.8 mg/ml when the O.D. 260 nm at the translational reaction of the embryo extract was 100.

The reaction solution prepared as mentioned above was reacted at 20°C. After a fixed time elapsed (10 minutes to 3 hours), the reaction solution was diluted using 2 to 3 volumes of the above translational reaction solution, then concentrated using an ultrafiltration membrane with a molecular weight cut-off at 30,000 Da to bring the reaction solution to the same volume as before the dilution. The operation was repeated for a plurality of times. Alternatively, it was concentrated to about 1/2 to 1/3 volume, then diluted using the translational reaction solution, to the original volume.

When the mRNA was to be additionally added into the translational reaction solution by a batch operation, the transcriptional reaction solution (before ethanol precipitation) that had been prepared in 1) or the mRNA solution that had been prepared through the ethanol precipitation operation, was added according to the concentration of the wheat embryo liquid.

### 4) Confirmation of the effect of synthesizing protein

The amount of synthesized protein was measured according to the method described in a report from Madin K et. al. (Madin K. et al., Proc. Natl. Acad. Sci. USA (2000), 97, 559-556).

### 5) Form of wheat embryo extract

The wheat embryo extract, the cell-free protein synthetic reaction liquid composition, the preparation method of mRNA, and the batch type method of synthesizing cell-free protein, used in the present examination were arranged according to the method described in the report from Madin K et al. (Madin K. et. al., Proc. Natl. Acad. Sci. USA (2000), 97, 559-556), Japanese Patent No. 3255784, Japanese Unexamined Patent Publication No. 2000-236896, WO00/68412, and the report from Sawasaki T. et. al. (Proc. Natl. Acad. Sci. USA (2002), 99, 14652-14657).

### (Example of apparatus)

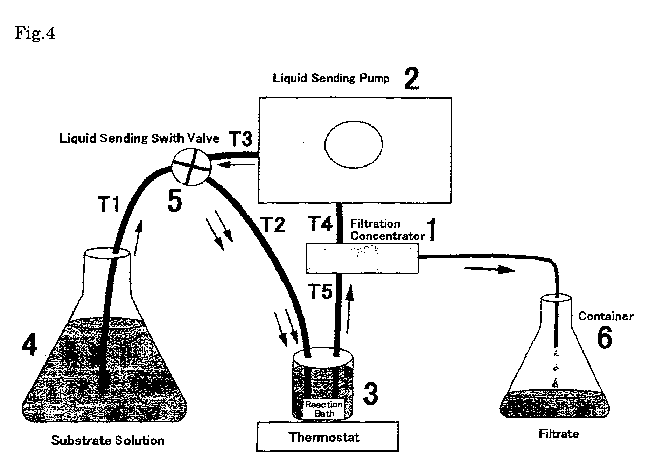

Fig. 4 shows a schematic diagram of a system wherein the dilution and the concentration operation of the present invention can be performed for a plurality of times, and for discontinuously performing the synthetic reaction. When the dilution and the concentration operation are achieved by a configuration having a filtration membrane and a liquid sending pump, then for example use of a mechanism as schematically shown in Fig. 4 is exemplified. By using a mechanism that is a combination of a filtration concentrator 1 having a filtration membrane, and a liquid sending pump 2, a (transcriptional or) translational reaction liquid is diluted with an (RNA) substrate solution around termination of the translational reaction in a reaction bath 3, and then the by-products are filtrated by the filtration concentrator 1 and discharged into a container 6. Meanwhile, the concentrated (transcriptional or) translational reaction liquid can be put back into the reaction bath 3 so as to restart the reaction.

In the system shown in Fig. 4, a container 4 containing the (RNA) substrate solution, and the reaction bath 3 are connected via a tube T1, a liquid sending switch valve 5, and a tube T2. The reaction bath 3 is connected to the liquid sending switch valve 5 via a tube T3, a liquid sending pump 2, a tube T4, the filtration concentrator 1, and a tube T5. In this manner, the configuration can appropriately switch between the following states: (a) a state where a liquid is not sent into the reaction bath 3 (a state where the valve is closed); (b) a state where the (RNA) substrate solution can be pumped up from the container 4 containing the (RNA) substrate solution and directly sent into the reaction bath 3 by the operation of the liquid sending pump 2; (c) a state where the (transcriptional or) translational reaction solution in the reaction bath 3 can be circulated by the operation of the liquid sending pump 2, so that the (transcriptional or) translational reaction solution is pumped up, passed through the filtration concentrator 1, then brought back into the reaction container 3; and (d) a state where the (RNA) substrate solution can be pumped up from the container 4 containing the substrate solution, passed through the filtration concentrator 1, and sent into the reaction bath 3 by the operation of the liquid sending pump 2.

The operation is performed by the following procedures using such a configuration.
1. The (transcriptional or) translational reaction is performed by a batch reaction in the reaction bath 3. In the meantime, the liquid sending switch valve 5 is closed [the state (a)] and the liquid sending pump 3 is also stopped so as to stop sending the liquid into the reaction bath 3.
2. At some point in time around stop of the (transcriptional or) translational reaction, the liquid sending switch valve 5 is switched over [state (b)] to operate the liquid sending pump 2 so as to supply the (RNA) substrate solution into the reaction bath 3 and mix therewith.
3. The liquid sending switch valve 5 is switched over [state (c) above] to operate the liquid sending pump 2 so as to circulate the (transcriptional or) translational reaction liquid in the reaction bath 3 so that it is pumped up, passed through the filtration concentrator 1, and the concentrated reaction liquid is brought back into reaction bath 3 while discharging by-products into the container 6.
4. The liquid sending switch valve 5 is switched over [state (d) above] to operate the liquid sending pump 2 so as to pump up the (RNA) substrate solution in the container 4, pass it through the filtration concentrator 1, and send it into the reaction bath 3, so that a part of the (transcriptional or) translational reaction liquid remaining in the filtration concentrator 1 is washed out and sent into the reaction bath 3.
5. The liquid sending switch valve 5 is switched over [state (b) above] to operate the liquid sending pump 2 so as to pump up the (RNA) substrate solution in the container 4, and send it into the reaction bath 3, so that the volume of the (transcriptional or) translational reaction liquid in the reaction bath 3 is prepared to the original volume. Then the liquid sending switch valve 5 is switched over [state (a) above] so as to stop sending the liquid.
6. The sequence then returns to 1 to repeat the operation.

The apparatus using such a system can be achieved by using for the liquid sending pump, a conventionally publicly known appropriate liquid sending pump without specific limitation. An example includes a peristaltic pump [for example, LKB-Pump-p1 (made by Pharmacia Corp.) and the like]. Moreover, a conventionally publicly known filtration concentrator may be used without specific limitation. A specific example includes a cross flow filtration unit VF05C2 (molecular weight cut-off at 30,000, made by Sartorius AG).

According to the system wherein the dilution and concentration means can be achieved by a configuration having the filtration membrane and the liquid sending pump, it becomes possible to constitute an apparatus wherein the protein synthetic yield can be increased (refer to experimental example 4) and the cell-free protein synthesis on a larger scale capacity can be performed, compared to a case where a centrifugal separator is used. Furthermore, by combining the small reaction container and the filtration concentrator, it is possible to constitute an apparatus of a small capacity. Therefore, the apparatus is not necessarily on large scale, differing from the aforementioned configuration having the filtration membrane and the centrifugal separator as the means for removing by-products. Moreover, in such a configuration having the filtration membrane and the liquid sending pump, the dilution and concentration process can be readily and accurately controlled. Therefore this configuration can be expected to become an extremely important key technique directing to automation of general cell-free protein synthesis technology corresponding to various objects.

### Experimental example 1

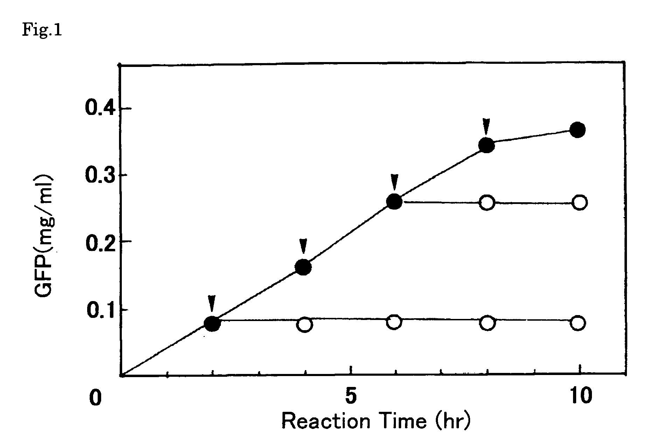

Fig. 1 shows experimental results using a wheat embryo extract, as an example (example 1) of a batch type method of synthesizing cell-free protein of the present invention wherein the dilution and concentration treatment is discontinuously repeated. The comparative example is the results by a conventional method of synthesizing cell-free protein by the batch method. The y axis shows the amount of the synthesized protein (mg/ml), and the x axis shows the reaction time (hr). In Fig. 1, the line linking the black circles (•-•) is the results of the present invention, and the line linking white circles (o-o) is the results of the conventional batch method. In the synthetic reaction, a wheat embryo extract at a concentration of 40A260 nm/ml (the absorbancy of wheat embryo extract at a wavelength of 260 nm) and 320 µg/ml of mRNA (translational template) that encodes Green fluorescent protein (GFP) were mixed, and the cell-free protein synthesis was performed at 20°C.

The synthetic reaction of the comparative example was performed using a concentrator with an ultrafiltration membrane with a molecular weight cut-off of 30,000 kDa. In the conventional batch reaction method, the time variation of the amount of the produced protein using the fluorescence activity as an index showed a typical hyperbolic shape. That is, the reaction velocity was decreased after passing the maximum velocity phase at the beginning of starting the reaction. After 3 hours when the reaction was coming to an end, an increase in the amount of synthesized protein was not found even if the temperature was kept warm for a long time (o-o). The results agree well with the reports heretofore (Madin K. et al., Proc. Natl. Acad. Sci. USA (2000), 97, 559-556). The cause of stopping the reaction has been not completely clarified, however it is considered to be that the reduction in ATP and GTP concentration mainly serving as the energy source (energy depletion), and the accumulation of inregenerative AMP and GMP serving as by-products, suppress the protein synthesis by some mechanism.

The synthetic reaction of the present invention was performed in a container with an ultrafiltration membrane. After 2 hours from the beginning of the reaction, when the reaction velocity was high and the reaction was coming to an end, a protein synthesis substrate solution (containing substrate and energy source) of 3 volumes with respect to the volume of the reaction liquid was added and mixed (dilution treatment). Next, centrifugation was performed by a centrifugal separator to concentrate this solution that had been diluted with the substrate solution, so as to bring it back to the original volume of the reaction solution before dilution (at the time points shown by the arrowheads) (concentration treatment). By discontinuously repeating the series of treatment of the dilution and concentration for a plurality of times, low molecular substances including by-products were removed through the ultrafiltration membrane and the concentration thereof was decreased, while the ATP, GTP, and amino acid that had been consumed in the synthetic reaction were recovered close to the respective concentrations at the beginning of the reaction. At the same time, ribosome derived from wheat embryo, tRNA, and all other translational factors were concentrated back to the original concentration at the beginning of the reaction, by the concentration treatment. By such treatment, it can be confirmed that the protein synthetic reaction that had been once decreased was restarted having the original high initial velocity, and then the reaction was decreased again (•-•). By discontinuously repeating such dilution and concentration treatment, it became possible to use the property of the time domain where the initial reaction velocity is high, thus achieving highly efficient cell-free protein synthesis. As shown in Fig. 1, by repeating the dilution and concentration treatment for 4 times, the synthetic amount of GFP which was 0.072 mg per 1 ml reaction capacity in the batch method could be increased to 0.41 mg. The amount of synthesized protein was calculated according to the GFP fluorescence intensity measurement technique (fluorescence peak wavelength 508 nm) assuming that the fluorescence intensity of 1 µg/ml GFP was 2.0.

The present treatment achieved sufficient synthetic efficiency. However, a phenomenon was found in that the system protein synthesis velocity gradually decreased with repetition of the dilution and concentration treatment. The reason was presumed to be due to a loss of the translational factors (group) derived from the embryo from within the reaction system, caused by leaking into the filtrate or absorption onto the ultrafiltration membrane; or to a decrease in the concentration of mRNA due to decomposition thereof or absorption onto the ultrafiltration membrane, and the like.

### Experimental example 2

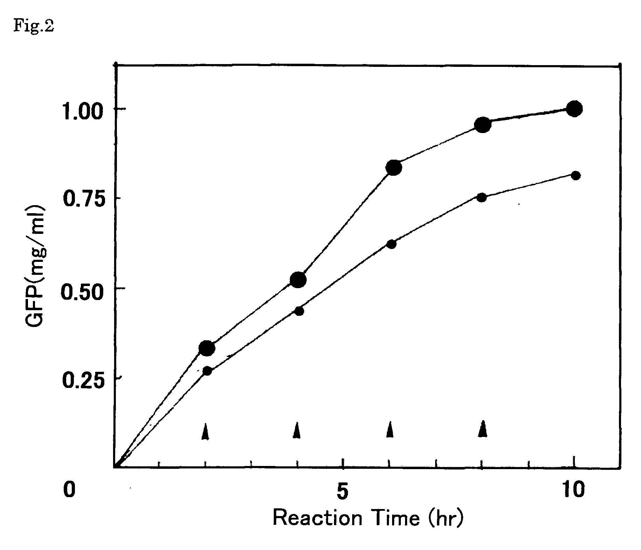

Fig. 2 is a graph showing experimental results of cell-free protein synthesis performed similarly to experimental example 1 except for the difference in the concentration of wheat embryo extract. The y axis shows the amount of the synthesized protein (mg/ml), and the x axis shows the reaction time (hr). In Fig. 2, the line linking the small black circles (•-•) shows the results in the case (example 2) where the concentration of the wheat embryo extract was set to 60A260 nm/ml and the concentration of GFP mRNA was set to 480 µg/ml. The line linking the large black circles (•-•) shows the results in the case (example 3) where the concentration of the wheat embryo extract was set to 80A260 nm/ml and the concentration of GFP mRNA was set to 640 µg/ml. Moreover, in Fig. 2, the arrowheads show the time point when the above operation was performed.

As shown in Fig. 2, in example 2, the amount of protein synthesized by repeating discontinuously the dilution and concentration treatment for 4 times in the cell-free system was 0.62 mg per 1 ml of the reaction liquid. In example 3, the amount of protein synthesized by repeating discontinuously the dilution and concentration treatment for 4 times in the cell-free system was 1.13 mg per 1 ml of the reaction liquid. In this manner, the protein synthetic amount by the method of synthesizing cell-free protein of the present invention was greater as the concentration of the translational factor in the reaction system becomes higher, and was greatly increased compared to the experimental results of the conventional batch method shown in Fig. 1.

### Experimental example 3

The time when the synthesis velocity is decreased in a normal batch method is dependent on the reduction of the substrate concentration in the reaction solution, and the concentration of the accumulated by-products. Therefore it becomes in inverse proportion to the concentration of the translational factor in the system. That is, as the concentration of the translational factor (concentration of the wheat embryo extract) in the system becomes higher, the time until the synthesis is stopped gets shorter (Madin K et. al. Proc. Natl. Acad. Sci. USA (2000), 97, 556-559). Therefore, if the translational factor is used at a high concentration, it is considered that a high synthesis velocity can be maintained by shortening the time interval for discontinuously repeating the dilution and concentration. As a result it can be expected to improve the efficiency of producing protein per unit time.

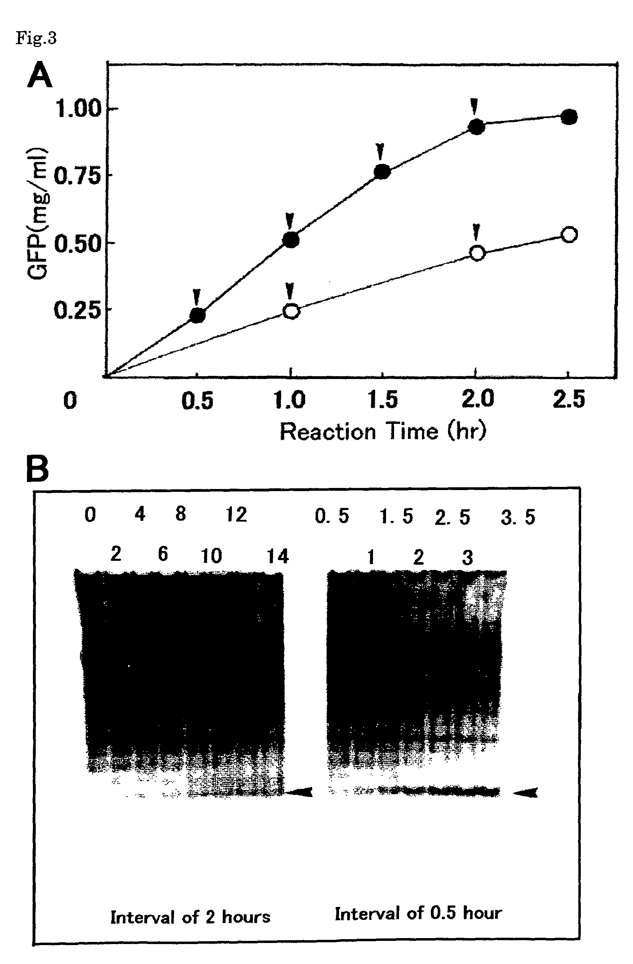

Fig. 3A is a graph showing the experimental results of cell-free protein synthesis performed similarly to experimental example 1 except for the difference in that the concentration of the wheat embryo extract was set to 80A260 nm/ml, the concentration of GFP mRNA was set to 640 µg/ml, and the time interval for discontinuously repeating the dilution and concentration was changed. Fig. 3B shows the results of polyacrylamide gel electrophoresis showing the difference in the amount of synthesized GFP depending on the difference in the interval for discontinuously repeating the dilution and concentration.

In the graph of Fig. 3A, the y axis shows the amount of synthesized protein (mg/ml), the x axis shows the reaction time (hr), and the arrowheads show the time point when the dilution and concentration was discontinuously repeated. In Fig. 3A, the line linking black circles (•-•) shows the experimental results in the case (example 4) where the dilution and concentration was discontinuously repeated at intervals of 0.5 hour from the beginning of the reaction. The line linking the white circles (o-o) shows the experimental results in the case (example 5) where the dilution and concentration was discontinuously repeated at intervals of 1 hour from the beginning of the reaction. In Fig. 3A, the arrowheads show the time point when the dilution and concentration was discontinuously repeated.

Fig. 3B shows a comparison between example 4 where the dilution and concentration was discontinuously repeated at interval of 0.5 hour, and the case (example 6) where the same procedures as those in example 4 were performed except that the dilution and concentration was discontinuously repeated at intervals of 2 hours. In Fig. 3B, the arrowheads show the GFP stained bands. In the polyacrylamide gel electrophoresis, 1 µl of the reaction liquid at the respective reaction times (time elapsed 0, 0.5, 1, 1.5, 2, 2.5, 3, 3.5 hours from the beginning of the reaction in example 4, time elapsed 0, 2, 4, 6, 8, 10, 12, 14 hours from the beginning of the reaction in example 6) was separated by non-denaturing polyacrylamide gel electrophoresis, and the protein was stained with Coomassie Brilliant Blue.

As shown in Figs. 3A and B, the yield per unit time in example 4 where the dilution and concentration was discontinuously repeated at intervals of 0.5 hour was higher than that of example 5 where the dilution and concentration was discontinuously repeated at intervals of 1 hour, and that of example 6 where the dilution and concentration was discontinuously repeated at intervals of 2 hours. In example 4, the obtained synthetic amount was 1.12 mg per 1 ml from the 2.5 hours reaction, which is more than the yield obtained from the 10 hours reaction by discontinuously repeating the dilution and concentration at intervals of 2 hours in the same concentration of the reaction liquid. The results directly show that as the concentration of the translational factor (the concentration of the wheat embryo extract) in the reaction system becomes higher, the concentration is reduced accompanying the consumption of the energy source over a short time, and at the same time the concentration of the accumulated by-product becomes higher, so that the time until the reaction velocity is decreased and the synthesis is stopped gets shorter. Consequently, when the time is set for discontinuously repeating the dilution and concentration, the maximum synthetic yield can be achieved by selecting an optimum timing depending on the concentration of the wheat embryo extract contained in the reaction system.

The general physical property of protein includes the instability (high activity). Consequently, one of the preconditions for obtaining high quality protein is to construct a technique for increasing the synthetic yield in a short time. The principle of the present invention which has been proven in Fig.3 can be said to be extremely useful.

### Experimental example 4

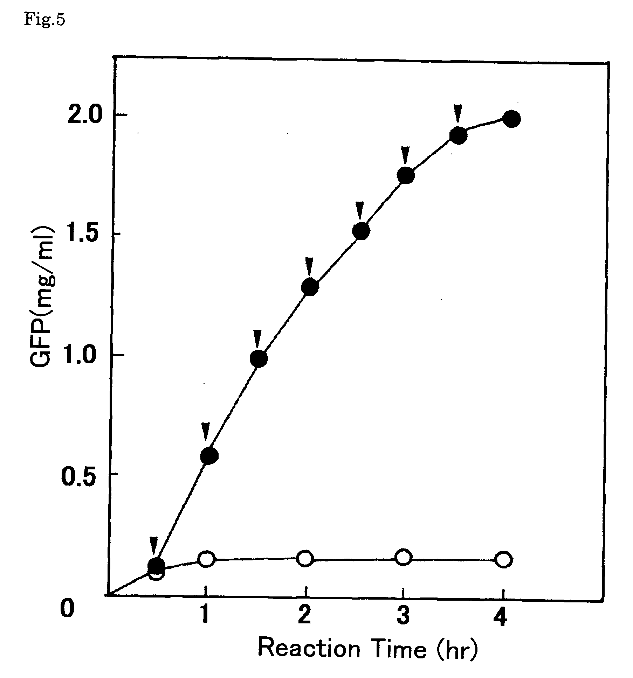

Fig. 5 is a graph showing experimental results (example 7) of cell-free protein synthesis of the present invention performed using the configuration shown in Fig. 4 where the filtration membrane and the liquid sending pump are used for removing by-products. The y axis shows the amount of the synthesized protein (mg/ml), and the x axis shows the reaction time (hour). In Fig. 5, the line linking the black circles (•-•) shows the experimental results of example 7 and the line linking the white circles (o-o) shows the experimental results of a method according to a conventional batch method. Moreover, in Fig. 5, the arrowheads show the time point when the dilution and concentration was discontinuously repeated.

In example 7, a cross flow filtration unit VF05C2 (molecular weight cut-off at 30,000, made by Sartorius AG) was used as the filtration concentrator, and a LKB-Pump-p1 (made by Pharmacia Corp.) was used as the liquid sending pump. Moreover, using a translational reaction solution similar to experimental example 1 except for the difference in that the concentration of the wheat embryo extract was set to 80A260 nm/ml, and the concentration of GFP mRNA was set to 640 µg/ml, the dilution and concentration was discontinuously repeated at intervals of 0.5 hour. A graduated plastic tube (Falcon tube) was used as the reaction container. The reaction volume was set to 15 ml. The dilution in the discontinuous repetition of the dilution and concentration was performed using 45 ml of the translational reaction solution.

As shown in Fig. 5, it was confirmed that when the configuration having the filtration membrane and the liquid sending pump was used as the device for removing by-products, this could be suitably used for the method of synthesizing cell-free protein of the present invention, similarly to the apparatus using the ultrafiltration membrane and the centrifugal separator in experimental examples 1 to 3. In example 7, 2.1 mg of protein was synthesized per 1 ml reaction volume from a 4 hours reaction, at a synthetic yield which was about two times higher than for the case of using the apparatus having the centrifugal separator and the ultrafiltration membrane.

### Experimental example 5

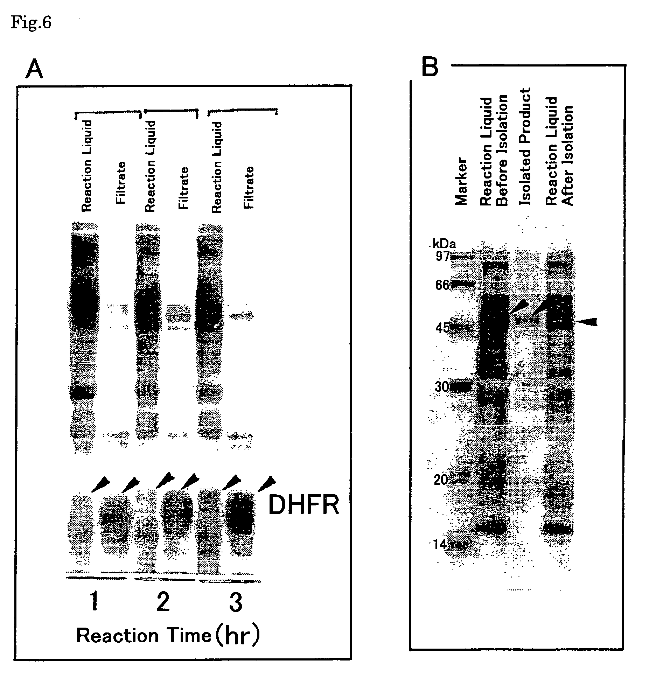

Fig. 6A is a graph showing the experimental results in the case (example 8) where the cell-free protein synthesis was performed similarly to example 1, except for the difference in that the concentration of the wheat embryo extract was set to 60A260 nm/ml, 450 µg/ml of mRNA of dihydrofolate reductase (DHFR) was used as the translational template, and the dilution and concentration was discontinuously repeated at intervals of 1 hour twice. For a comparative example, the cell-free protein was synthesized under similar conditions except for using a conventional batch method (the discontinuous repetition of dilution and concentration was not performed). In example 8, when synthesizing DHFR of molecular weight of about 20,000 KDa in a cell-free system, a reaction container with an ultrafiltration membrane with a molecular weight cut-off at 30,000 Da (made by Millipore Corporation) was used.

In Fig. 6A, lanes 1, 2, and 3 respectively show the discharged solutions (filtrate) after the reaction by the discontinuous repetition of the dilution and concentration for 0, 1, and 2 times in experiment 8, and the reaction liquids by the conventional batch method at time points similar to those for the discontinuous repetition of the dilution and concentration, each 1 µl of which was subjected to sodium dodecyl sulfate-polyacrylamide gel electrophoresis (SDS-PAGE) and stained with Coomassie Brilliant Blue (the arrowheads in the drawing show the DHFR being the synthesized product). As understood from the strength of DHFR stained bands shown by the arrowheads in Fig. 6A, only a trace of DHFR synthesis was confirmed after 1 hour of the reaction in the experimental results according to the conventional batch method ("reaction liquid" on the left of each lane). However it was confirmed that the DHFR synthesized in the reaction system by the discontinuous repetition of the dilution and concentration for once or twice could be efficiently taken out into the filtrate in the experimental results of example 8 ("filtrate" on the right of each lane).

### Experimental example 6

Fig. 6B is an electrophoretogram showing the results of an experiment in which GFP fusion protein having streptavidin fused on the N-terminus was synthesized by the method of synthesizing cell-free protein of the present invention (example 9), and the GFP fusion protein was isolated from the system.

The cell-free protein synthesis itself in the present experiment was performed similarly to example 1, except for the difference in that the mRNA encoding the GFP fusion protein was used as the translational template, and the dilution and concentration was discontinuously repeated at intervals of 1 hour for 4 times. The operation to isolate the GFP fusion protein from the system was performed in a procedure where: 1) magnetic beads immobilized with biotin were added into the reaction solution, and the generated fusion product was selectively collected utilizing the affinity between biotin and streptavidin; and 2) the beads bonded with the fusion protein were taken out from the reaction container by a magnet. Moreover, the electrophoresis and staining were performed similarly to experimental example 5.

As shown in Fig. 6B, it was found that the product that had been detected in the reaction liquid before the isolation, has disappeared from the reaction liquid due to the present operation (arrow head on the lane of the reaction liquid after isolation), and been efficiently isolated from the system through the magnetic beads (synthesized product shown in the lane of the isolated product).

### Experimental example 7

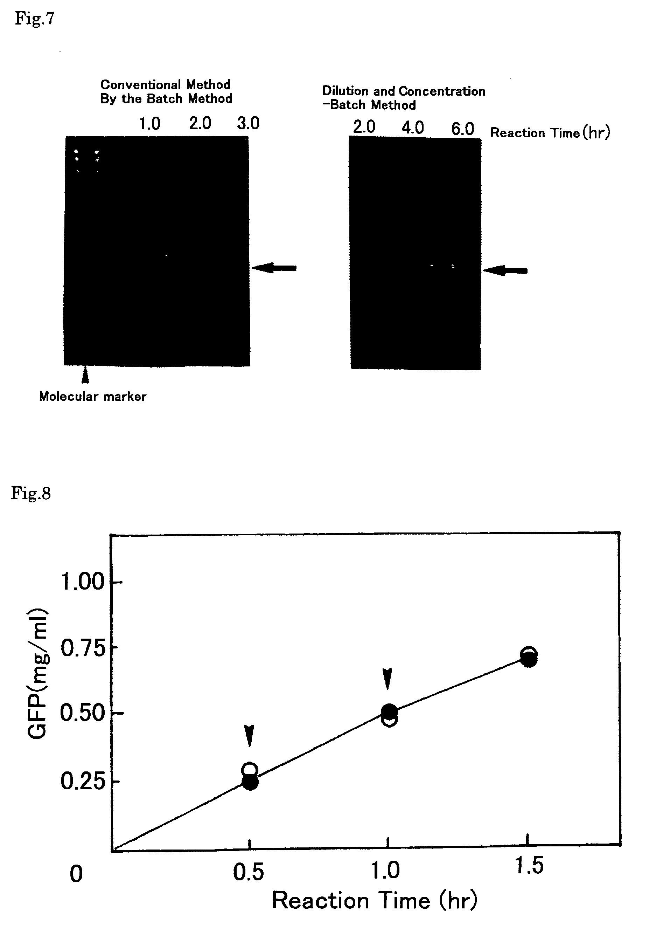

Fig. 7 shows a difference in the experimental results between the method of synthesizing RNA of the present invention (example 10), and a conventional method of synthesizing RNA in vitro by the batch method. In Fig. 7, the left shows the results of the conventional method by the batch method, the right shows the results of the method of synthesizing RNA of the present invention, and the arrows show the mRNA product encoding GFP.

Respectively using a jellyfish GFP gene that had been inserted into a plasmid vector pEU exclusive for a wheat embryo cell-free system, as the transcriptional template, the transcriptional reaction solution [final concentration: 80 mM HEPES-KOH pH 7.8, 16 mM magnesium acetate, 10 mM dithiothreitol, 2 mM spermidine, 2.5 mM 4NTPs (4 types of nucleotide-triphosphate), 0.8 U/µl RNase inhibitor, 0.1 µg/µl DNA [plasmid GFP (Green fluorescent protein)], and 1.6 U/µl SP6 RNA polymerase] was prepared and reacted at 37°C for 3 hours.

Regarding the RNA synthetic reaction in example 10, 3 volumes of the solution for transcriptional reaction was added into the transcriptional reaction liquid after 3 hours from the beginning of the reaction, to perform the dilution, and then low-molecular substances were removed from the reaction liquid system through the filtration membrane using the centrifugal separator, so as to concentrate the transcriptional reaction liquid to the original concentration. Then, the dilution and concentration was discontinuously repeated at intervals of 3 hours to progress the synthetic reaction. When the dilution and concentration was being discontinuously repeated, the transcriptional reaction liquid was dispensed, and the transcription product (mRNA) that had been separated by the agarose gel electrophoresis was visualized by ethidium bromide staining.

As shown in Fig. 7, in the conventional method of synthesizing RNA in vitro by the batch method, the RNA synthetic reaction was stopped for about 3 hours. However in example 10 where the dilution and concentration was discontinuously repeated after 3 hours of reaction having a high synthesis velocity, the mRNA could be synthesized at a yield of 2.4 times greater than that of the conventional method.

### Experimental example 8

Fig. 8 is a graph showing the experimental results of the method of synthesizing cell-free protein of the present invention performed using mRNA purified by ethanol precipitation, and unpurified mRNA respectively as the translational template. The y axis shows the amount of the synthesized protein (mg/ml), and the x axis shows the reaction time (hour). The purified mRNA was prepared according to the method described in example (method of synthesizing cell-free protein) 1) reaction from DNA into mRNA (transcriptional reaction). The unpurified mRNA was prepared by the method described in example 10, then centrifuged at 12,000 rpm for 1 hour, and the supernatant was collected and prepared. In Fig. 8, the line linking the black circles (•-•) shows the experimental results in the case (example 11) where the protein synthetic reaction was performed by adding the transcriptional reaction liquid containing mRNA that had been synthesized in example 10, into the translational reaction liquid at a ratio of 1:2, then after mixing, adding the same volume as the mixed liquid, of dialytic buffer [35 mM HEPES-KOH pH 7.8, 103 mM potassium acetate (KOAc), 3.1 mM magnesium acetate (Mg(OAc)₂), 16 mM creatine phosphate, 1.2 mM ATP, 0.26 mM GTP, 2.5 mM dithiothreitol (DTT), 0.43 mM spermidine, 0.3 mM AAs (mixture of 20 types of L-amino acid)] to dilute the liquid, and then concentrating so as to bring the volume back to the original volume of the mixture, and then again adding the same volume of the dialytic buffer, and then repeating the same process again to concentrate, and then adding the creatine kinase so that the final concentration became about 1 mg/ml. The line linking the white circles (o-o) shows the experimental results in a case (example 12) where the protein synthetic reaction was performed with a translational reaction liquid prepared using the mRNA synthesized in experimental example 7 followed by purification by ethanol precipitation. Examples 11 and 12 were performed similarly to example 4 except for the difference in that the concentration of mRNA was set to 480 µg/ml, and the concentration of the wheat embryo extract was set to 60A260 nm/ml.

As shown in Fig. 8, no significant difference was found in the protein synthesis velocities between examples 11 and 12. The result showed that, according to the method of synthesizing cell-free protein of the present invention, it becomes possible to realize a simple method of synthesizing cell-free protein performed using the translational reaction liquid that is prepared using the transcriptional reaction liquid after transcription, without purifying mRNA.

### Experimental example 9

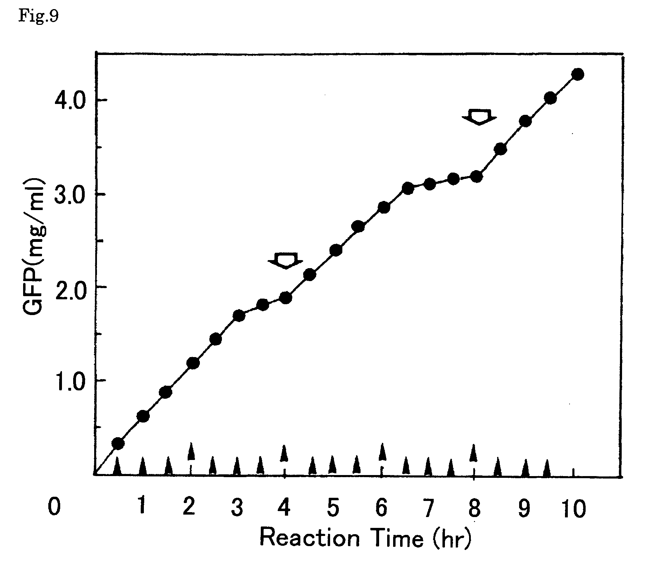

Fig. 9 is a graph showing the experimental results of the method of synthesizing cell-free protein of the present invention performed with the translational reaction liquid that had been prepared using the transcriptional reaction liquid after the transcriptional reaction of experimental example 7. The y axis shows the amount of the synthesized protein (mg/ml), and the x axis shows the reaction time (hour). In Fig. 9, the upward arrowheads show the time points when the dilution and concentration was discontinuously repeated. The downward arrowheads show the time points when the transcriptional reaction liquid (including mRNA) after the transcriptional reaction was added.

The translational reaction liquid was prepared by adding 80A260 nm/ml of wheat embryo extract and the transcriptional reaction liquid (including GFP mRNA 9.6 mg) after the transcriptional reaction, into 15 ml of the solution for translational reaction that was similar to example 1 (final concentration of GFP mRNA was 640 µg/ml), which was then concentrated to a liquid volume of 8 ml, and dilution using the solution for translational reaction and concentration were performed. Then, the translational reaction was started. As a device for removing by-products, similarly to Fig. 4, a configuration having the filtration membrane and the liquid sending pump was used, the GFP mRNA (translational template) was added every 4 hours from the beginning of the reaction, and the dilution and concentration was discontinuously repeated at intervals of 30 minutes by using a solution containing the substrate and energy source.

As shown by the experimental results, it can be confirmed that the translational reaction that had been substantially stopped can be restarted by adding mRNA. Furthermore, it can be understood that the protein synthesis can be maintained for a long time by combining the dilution/concentration method and mRNA addition.

### Embodiments of the apparatus

Hereunder is a description of embodiments of the automatic synthesizer according to the present invention. However, the automatic synthesizer according to the present invention is not intended to be limited to the apparatus of the following embodiments provided it can automatically executes the high throughput synthesis system.

In order to run the automatic synthesizer of the present invention, the following were respectively set into the automatic synthesizer, and the GFP protein synthesis was performed using the synthesizer.

### Transcriptional template

DNA obtained from PCR in a 30 µl system using a pEU plasmid vector inserted with GFP and a sense primer and an antisense primer was used as the transcriptional template. The DNA was put into a 96- well PCR plate (plate for template: (1)).

### Solution for transcriptional reaction

4.95 ml of a solution containing the respective final concentration of: 80 mM HEPES-KOH, 16 mM magnesium acetate, 2 mM spermidine, 10 mM DTT, 3 mM NTPs, 1 U/µl SP6 RNA polymerase, and 1 U/µl RNasin was prepared and put into a reagent bath 1 (2) in the apparatus.

### Solution for translational reaction

5.5 ml of solution containing as the final concentration: 30 mM HEPES-KOH (pH 7.8), 1.2 mM ATP, 0.25 mM GTP, 16 mM phosphocreatine, 2 mM dithiothreitol, 0.3 mM spermidine, 0.3 mM 20 types of amino acids, 2.7 mM magnesium acetate, 100 mM potassium acetate, 0.005% sodium azide, 40 ng/µl creatine kinase and wheat embryo extract of 80 or 40 units with an optical density (O.D.) at 260 nm, was prepared and put into a reagent bath 2 (3) in the apparatus. The protein synthesis using the solution for translational reaction containing respectively 80 or 40 units of wheat embryo extract was separately performed.

### Diluting solution

100 ml of a solution containing as the respective final concentration of: 30 mM HEPES-KOH (pH 7.8), 1.2 mM ATP, 0.25 mM GTP, 16 mM phosphocreatine, 2 mM dithiothreitol, 0.3 mM spermidine, 0.3 mM 20 types of amino acids, 2.7 mM magnesium acetate, 100 mM potassium acetate, and 0.005% sodium azide, was prepared and put into a reagent bath 3 (4) in the apparatus.

### Synthesis container

A plate for the template (lidded PCR 96-well (length 8 x width 12) plate (containing template DNA): (1)), a plate for transcription/filtrate reception (lidded 96-well (length 8 x width 12), a titer plate: (10)), a plate for translation (lidded PCR 96-well (length 8 x width 12) plate (filter at the bottom of the cell): (14)), and a dummy plate (for balancing during the centrifugation).

### Tips

280 of 300 µl tips (for solution for transcriptional reaction, for solution for translational reaction, and for diluting solution: (5)), 96 of 20 µl tips (for template product: (6))
dispenser 1: 8 pipetters (for attaching 300 µl tip: (8))
dispenser 2: 8 pipetters (for attaching 20 µl tip: (9))

The GFP protein synthesis was performed in the following steps using the synthesizer according to the present invention.

### <Step 1: Transcription>

(1) The robot arm (7) removed the lid of the plate for transcription/filtrate reception (10).
(2) The dispenser 1 (8) was attached with the 300 µl tips (5) and moved to the reagent bath 1 (2) containing the transcriptional reaction liquid.
(3) The dispenser 1 (8) drew 95 µl (45 µl x 2 times + 5 µl) of the transcriptional reaction liquid from the reagent bath 1 (2).
(4) The dispenser 1 (8) was moved to the position of the plate for transcription/filtrate reception (10), and discharged 45 µl of the transcriptional reaction liquid twice into (8 of) the respective lengthwise cells in the plate.
(5) The steps (2) to (4) were further performed for the respective lengthwise cells in the 7 rows not yet containing the transcriptional reaction liquid.
(6) The dispenser 1 (8) was moved to the position of the waste tip opening (11) and disposed the tips.
(7) The robot arm (7) removed the lid of the plate for the template (1).
(8) The dispenser 2 (9) was attached with the 20 µl tips (6).
(9) The respective tips of the dispenser 2 (9) drew 5 µl of air (for increasing the drawing efficiency).
(10) The dispenser 2 (9) was moved to the position of the plate for the template (1), and drew 5 µl of the template sample in (8 of) the respective lengthwise cells in the plate for the template (1).
(11) The dispenser 2 (9) was moved to the position of the plate for transcription/filtrate reception (10), and discharged the whole amount of the template sample into the (8 of) the respective lengthwise cells in the plate.
(12) The (8 of) the respective lengthwise cells in the plate for transcription/filtrate reception (10) were mixed by pipetting of the respective tips of the dispenser 2 (9).
(13) The dispenser 2 (9) was moved to the position of the waste tip opening (11) and disposed the tips.
(14) The steps (8) to (13) were further performed for the respective lengthwise cells in the 7 rows not yet containing the template sample.
(15) The robot arm (7) put the lid on the plate for the template (1) and the plate for transcription/filtrate reception (10).
(16) The robot arm (7) transferred the plate for transcription/filtrate reception (10) to the constant temperature bath 1 (12) which had been previously set at 37°C, and set it therein.
(17) The transcriptional reaction was performed for 4 hours at 37°C in the constant temperature bath 1 (12)

### <Step 2: Removal of precipitation after transcription>

(1) The robot arm (7) transferred the plate for transcription/filtrate reception (10) from the constant temperature bath 1 (12) onto the MTP stage (13).
(2) The robot arm (7) removed the lid of the plate for transcription/filtrate reception (10).
(3) The dispenser 1 (8) was attached with the 300 µl tips (5).
(4) The respective tips of the dispenser 1 (8) drew 5 µl of air.
(5) The dispenser 1 (8) was moved to the reagent bath 3 (4) containing the diluting solution, and drew the diluting solution (the drawn amount was finely adjusted between 80 µl to 120 µl for each row since the liquid volume after the concentration centrifugation is variable according to the row in the plate).
(6) The dispenser 1 (8) was moved to the position of the plate for transcription/filtrate reception (10), and discharged the whole amount of the diluting solution into the (8 of) the respective lengthwise cells in the plate.
(7) The steps (4) to (6) were further performed for the respective lengthwise cells in the 7 rows not yet containing the diluting solution.
(8) The dispenser 1 (8) was moved to the position of the waste tip opening (11) and disposed the tips.
(9) The robot arm (7) put the lid on the plate for translation (14) and laid it over the plate for transcription/filtrate reception (10) (in order to balance with the dummy plate during the centrifugation).
(10) The robot arm (7) transferred the laid plate for translation (14) and the plate for transcription/filtrate reception (10) onto the lifting/lowering platform (15).
(11) The lifting/lowering platform on which the laid plate for translation (14) and the plate for transcription/filtrate reception (10) were mounted, was lowered so as to match the height of the centrifugal separator (16).
(12) The robot arm (7) opened the door of the centrifugal separator (16).
(13) The centrifugal separator (16) performed the position adjustment of the plate setting part on the centrifugal separator so as to set the plate.
(14) The robot arm (7) transferred the laid plate for translation (14) and the plate for transcription/filtrate reception (10) into the centrifugal separator, and set it therein.
(15) The position adjustment was performed similarly to (13), and the dummy plate was set into the centrifugal separator for balancing.
(16) The centrifugation was performed by the centrifugal separator (16) at 3100 g for 15 minutes.

### <Step 3: Transcription liquid buffer exchange first time>

(1) After the centrifugal separator (16) had been stopped, the robot arm opened the door of the centrifugal separator, then the centrifugal separator performed the position adjustment (position adjustment of the robot arm and the plate setting part on centrifugal separator).
(2) The robot arm (7) transferred the laid plate for translation (14) and the plate for transcription/filtrate reception (10) from the centrifugal separator (16) onto the lifting/lowering platform.
(3) The position adjustment was performed similarly to (1), and the robot arm (7) transferred the dummy plate onto the lifting/lowering platform.
(4) The robot arm (7) transferred the laid plate for translation (14) and the plate for transcription/filtrate reception (10) onto the MTP stage (13), and separately set the plate for translation (14) and the plate for transcription/filtrate reception (10) thereon.
(5) The robot arm (7) removed the lid of the plate for translation (14).
(6) The dispenser 1 (8) was attached with the 300 µl tips (5).
(7) The respective tips of the dispenser 1 (8) drew 5 µl of air.
(8) The dispenser 1 (8) was moved to the position of the plate for transcription/filtrate reception (10), and drew the whole amount of the liquid in the (8 of) the respective lengthwise cells in the plate for transcription/filtrate reception (10).
(9) The dispenser 1 (8) was moved to the position of the plate for translation (14), and discharged the whole amount of the liquid into the (8 of) the respective lengthwise cells in the plate for translation (14).
(10) The steps (6) to (9) were further performed for the respective lengthwise cells in the other 7 rows.
(11) The dispenser 1 (8) put the lid on the plate for translation (14).
(12) The dispenser 1 (8) laid the plate for translation (14) over the plate for transcription/filtrate reception (10) (top: plate for translation, bottom: plate for transcription/filtrate reception).
(13) The centrifugation was performed similarly to step 2 (10) to (16).

### <Step 4: Transcription liquid buffer exchange second time>

(1) Similarly to step 3 (1) to (5), the plate for transcription/filtrate reception and the plate for translation were taken out from the centrifugal separator and set onto the MTP stage, then the lid of the plate for translation was removed.
(2) The dispenser 1 (8) was attached with the 300 µl tips (5).
(3) The respective tips of the dispenser 1 (8) drew 5 µl of air.
(4) The dispenser 1 (8) was moved to the reagent bath 3 (4) containing the diluting solution.
(5) The respective cells in the dispenser 1 (8) drew the diluting solution (the drawn amount was finely adjusted between 80 µl to 120µl for each row).
(6) The dispenser 1 (8) was moved to the position of the plate for translation (14), and discharged the whole amount of the liquid into the (8 of) the respective lengthwise cells in the plate for translation (14).
(7) The steps (3) to (6) were further performed for the respective lengthwise cells in the other 7 rows.
(8) The dispenser 1 (9) was moved to the position of the waste tip opening (11) and disposed the tips.
(9) The dispenser 1 (8) was attached with the 300 µl tips (5).
(10) The respective tips of the dispenser 1 (8) drew 5 µl of air.
(11) The dispenser 1 (8) was moved to the plate for translation (14).
(12) The (8 of) the respective lengthwise cells in the plate for translation (14) were mixed by pipetting of the respective tips of the dispenser 1 (8).
(13) The steps (8) to (12) were further performed for the respective lengthwise cells in the other 7 rows (tips might be exchanged in the respective cells in some cases).
(14) The dispenser 1 (9) was moved to the position of the waste tip opening (11) and disposed the tips.
(15) The robot arm (7) put the lid on the plate for translation (14).
(16) The dispenser 1 (8) was attached with the 300 µl tips (5).
(17) The respective tips of the dispenser 1 (8) drew 5 µl of air.
(18) The dispenser 1 (8) was moved to the position of the plate for transcription/filtrate reception (10).
(19) The dispenser 1 (8) drew the whole amount of the liquid in the (8 of) the respective lengthwise cells in the plate for transcription/filtrate reception (10).
(20) The dispenser 1 (8) was moved to the position of the waste liquid opening (17).
(21) The dispenser 1 (8) discharged the whole amount of the solution into the waste liquid opening (17).
(22) The steps (17) to (21) were further performed for the respective lengthwise cells in the other 7 rows.
(23) The dispenser 1 (9) was moved to the position of the waste tip opening (11) and disposed the tips.
(24) The plate for translation (14) was centrifuged similarly to step 2 (9) to (16).

### <Step 5: Dispensation of solution for translational reaction>

(1) Similarly to step 3 (1) to (5), the plate for transcription/filtrate reception and the plate for translation were taken out from the centrifugal separator and set onto the MTP stage, then the lid of the plate for translation (14) was removed.
(2) The dispenser 1 (8) was attached with the 300 µl tips (5), and the respective tips drew 5 µl of air.
(3) The dispenser 1 (8) was moved to the reagent bath 2 (3) containing the solution for translational reaction.
(4) The dispenser 1 (8) drew 50 µl of the solution for translational reaction from the reagent bath 2 (3).
(5) The dispenser 1 (8) was moved to the position of the plate for translation (14), and discharged the whole amount of the solution into the (8 of) the respective lengthwise cells in the plate for translation (14).
(6) The (8 of) the respective lengthwise cells in the plate for translation (14) were mixed by pipetting of the respective tips of the dispenser 1 (8).
(7) The steps (2) to (6) were further performed for the respective lengthwise cells in the other 7 rows.
(8) The dispenser 1 (9) was moved to the position of the waste tip opening (11) and disposed the tips.
(9) The robot arm (7) put the lid on the plate for translation (14).
(10) The robot arm (7) transferred the plate for translation (14) to the constant temperature bath 1 (12).
(11) The plate for translation (14) was kept warm at 26°C for 1 hour.
(12) During the warming step of (11) (hereafter, step for disposing filtrate), the dispenser 1 (8) was attached with the 300 µl tips (5), and the respective tips drew 5 µl of air.
(13) The dispenser 1 (8) was moved to the position of the plate for transcription/filtrate reception (10).
(14) The dispenser 1 (8) drew the whole amount of the liquid in the (8 of) the respective lengthwise cells in the plate for transcription/filtrate reception (10).
(15) The dispenser 1 (8) was moved to the position of the waste liquid opening (17), and discharged the whole amount of the liquid.
(16) The steps (12) to (15) were further performed for the respective lengthwise cells in the other 7 rows.
(17) The dispenser 1 (9) was moved to the position of the waste tip opening (11) and disposed the tips.

### <Step 6: Repetition of translation>

The following steps were repeated for 6 times.
(1) The robot arm (7) transferred the plate for translation (14) from the constant temperature bath 1 (12) onto the MTP stage (13).
(2) The robot arm (7) removed the lid of the plate for translation (14).
(3) The dispenser 1 (8) was attached with the 300 µl tips (5).
(4) The respective tips of the dispenser 1 (8) drew 5 µl of air.
(5) The dispenser 1 (8) was moved to the reagent bath 3 (4) containing the diluting solution, and drew the diluting solution (the drawn amount was finely adjusted between 80 µl to 120 µl for each row since the liquid volume after the centrifugation is variable according to the row in the plate).
(6) The dispenser 1 (8) was moved to the position of the plate for translation (14) and discharged the whole amount of the diluting solution into the (8 of) the respective lengthwise cells in the plate.
(7) The steps (4) to (6) were further performed for the respective lengthwise cells in the 7 rows not yet containing the diluting solution.
(8) The centrifugation was performed similarly to steps 2 (9) to (16) (centrifugation time was 18 minutes).
(9) Similarly to step 3 (1) to (5), the plate for transcription/filtrate reception and the plate for translation were taken out from the centrifugal separator and set onto the MTP stage, then the lid of the plate for translation was removed.
(10) The dispenser 1 (8) was attached with the 300 µl tips (5), and the respective tips drew 5 µl of air.
(11) The dispenser 1 (8) was moved to the plate for translation (14).
(12) The (8 of) the respective lengthwise cells in the plate for translation (14) were mixed by pipetting of the respective tips of the dispenser 1 (8).
(13) The dispenser 1 (9) was moved to the position of the waste tip opening (11) and disposed the tips (the tips might not be exchanged in some cases).
(14) The steps (10) to (13) were further performed for the respective lengthwise cells in the other 7 rows
(15) The robot arm (7) put the lid on the plate for translation (14).
(16) The robot arm (7) transferred the plate for translation (14) to the constant temperature bath 1 (12).
(17) The plate for translation (14) was kept warm at 26°C for 1 hour.
(18) During the warming step of (17) (hereafter, step for disposing filtrate), the dispenser 1 (8) was attached with the 300 µl tips (5), and the respective tips drew 5 µl of air.
(19) The dispenser 1 (8) was moved to the position of the plate for transcription/filtrate reception (10).
(20) The dispenser 1 (8) drew the whole amount of the liquid in the (8 of) the respective lengthwise cells in the plate for transcription/filtrate reception (10).
(21) The dispenser 1 (8) was moved to the position of the waste liquid opening (17).
(22) The dispenser 1 (8) discharged the whole amount of the liquid.
(23) The steps (18) to (22) were further performed for the respective lengthwise cells in the other 7 rows.

### <Step 7: Finish (after performing step 6 for 6 times)>

(1) After finishing the warming of step 6 (18), the temperature of the thermostat was set to 4°C.
(2) The plate for translation was taken out from the synthesizer.

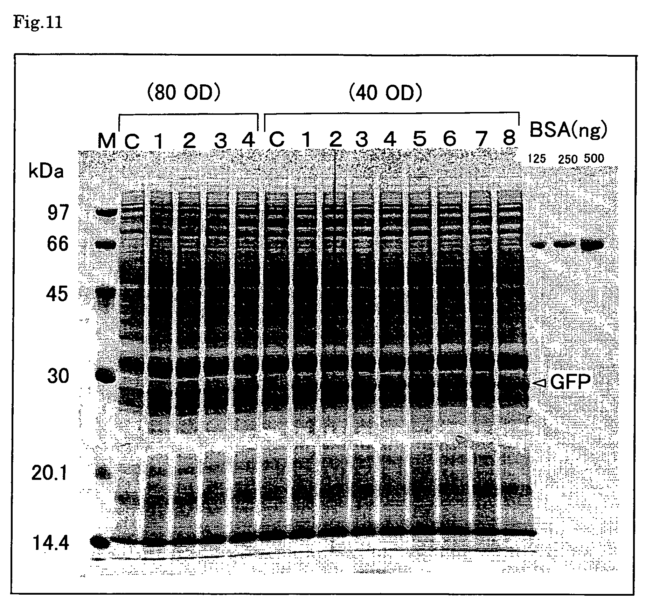

The amount of the synthesized protein of GFP synthesized in the above steps was measured according to the method described in the report of Madin K et. al. (Madin K. et. al., Proc. Natl. Acad. Sci. USA (2000), 97, 559-556), and analyzed by SDS-PAGE (Fig. 11).

In Fig. 11, regarding all 1-8 lanes of 40 units and 1-4 lanes of 80 units, the protein was synthesized at the positions of the GFP band. Furthermore, comparing the band concentration of BSA (125, 250, 500 ng) and the band concentration of GFP on each lane, it can be suggested that at least 250 ng of GFP was synthesized in all lanes.

As is apparent from the above description, it is understood that the high throughput synthesis system of the present invention which selects the reaction initial phase having the high reaction velocity, and consists of discontinuous repetition of dilution and concentration, and the apparatus which automatically performs this system, can synthesize high quality protein efficiently in a short time, and are also extremely useful for isolating the target protein. Moreover, it is understood that the principle is useful for efficiently synthesizing RNA in vitro.

Furthermore, it has been shown that the protein and the RNA synthesis method applying this principle, can solve a reaction apparatus caused by the various shortcomings that have been found in the Spirin's continuation method, that is, the complexity as an apparatus, the low strength of the membrane, clogging of the membrane during the operation, the complexity as a method and the like. In addition, the shortcoming of the continuation method in that the synthetic reaction takes a long time has remained as a big problem which must be resolved, not merely from the viewpoint of waste of time, but also from the viewpoint of securing the quality of the protein produced. The shortcoming has been unsolved problem in the double layer method.

### Industrial Applicability

The technology invented here could provide the basic elemental technology towards protein research in the post genomic era. In particular, it can be said to be essential as elemental technology for enabling the simple and efficient comprehensive preparation and mass production for analyzing the structure and the function of RNA, protein, and the like.

## Claims

1. A cell-free synthesis system wherein a template substance is used as a raw material, including the following systems, a control system therefor, or combinations thereof;
1) to make a template substance, a substrate, and a reaction solution into contact so as to lead to a synthetic reaction,
2) to perform dilution treatment of the reaction solution approximately around the decrease in the synthesis velocity, approximately around the stop of the synthetic reaction, or in the middle thereof,
3) to perform concentration treatment subsequent to the dilution treatment, and
4) to perform synthetic reaction by the concentrated reaction system,
or
1) to make a template substance, a substrate, and a reaction solution into contact so as to lead to a synthetic reaction,
2) to perform concentration treatment of the reaction solution approximately around the decrease in the synthesis velocity, approximately around the stop of the synthetic reaction, or in the middle thereof,
3) to perform dilution treatment subsequent to the concentration treatment, and
4) to perform synthetic reaction by the diluted reaction system.

2. The system according to claim 1 wherein by-products are removed from the reaction system in the concentration treatment.

3. The system according to claim 1 or 2, wherein the substrate, an energy source, and/or the template substance is replenished into the reaction system in the dilution treatment.

4. The system according to claim 1, wherein the systems of 1) to 4) are repeated for a plurality of times.

5. The system according to any one of claims 1 to 4, wherein the template substance is a transcriptional template.

6. The system according to any one of claims 1 to 4, wherein the template substance is a translational template.

7. The system according to claim 6, wherein the reaction solution contains a cell extract.

8. A cell-free synthesis system wherein a transcription product production system obtained from the system of claim 5, and a translation product production system obtained from the system of claim 6 or 7 are combined.

9. A synthetic kit including at least one reagent used for the cell-free system synthetic method using a template substance, which uses the system according to any one of claims 1 to 8.

10. A cell-free system synthetic method using a template substance, which uses the system according to any one of claims 1 to 8.

11. A cell-free system synthesizer using a template substance, which uses the system according to any one of claims 1 to 8.

12. A cell-free system synthesizer for automatically executing the system according to any one of claims 1 to 8, comprising at least the following control systems for executing a plurality of times a synthesis system using the initial phase in a synthetic reaction having a high protein synthetic reaction velocity;
(1) a system for starting the synthesis;
(2) a system for concentrating the reaction liquid;
(3) a system for diluting the reaction liquid;
(4) a system for reactivating the synthetic reaction; and
(5) a system for repeating the operations of the systems of (2) to (4);
or
(1) a system for starting the synthesis;
(2) a system for diluting the reaction liquid;
(3) a system for concentrating the reaction liquid;
(4) a system for reactivating the synthetic reaction; and
(5) a system for repeating the operations of the systems of (2) to (4).

13. The synthesizer according to claim 12, wherein the system for concentrating the reaction liquid uses an ultrafiltration membrane and, when the reaction liquid is removed from the reaction system through the ultrafiltration membrane, substances in the reaction liquid which can not pass through the ultrafiltration membrane are concentrated in the reaction system.

14. The synthesizer according to claim 13, wherein a centrifugal separator and/or a suction pump is used for removing the reaction liquid from the reaction system in the system for concentrating the reaction liquid.

15. The synthesizer according to claim 12, wherein the system for diluting the reaction liquid is to add a diluting solution or a substrate solution into the reaction liquid.

16. The synthesizer according to any one of claims 11 to 15, at least comprising, in a step for synthesizing a cell-free protein, one of the following control systems for automatically performing the step until the protein encoded by the template is produced from the transcriptional template;
(1) a system which variably controls the temperature in the reaction container;
(2) a system which dispenses a sample or a reagent into the reaction container;
(3) a system which transfers the reaction container; and
(4) a system for precipitation and concentration filtration.

17. A program for executing the system according to any one of claims 1 to 8, including the following information processing means for executing a plurality of times a synthesis system using the initial phase in a synthetic reaction having a high protein synthetic reaction velocity;
(1) an information processing means for setting the synthesis time within a range of an initial phase in the synthetic reaction before the synthetic amount per unit time tends to decrease, based on information on the volume of the reaction container and the concentration of the reaction liquid,
(2) an information processing means for setting the reaction liquid out of a range of the concentration in which the synthetic reaction is substantially possible, by adding a diluting solution into the reaction container when the time comes up to the synthesis time within the range of the initial phase in the synthetic reaction of (1),
(3) an information processing means for concentrating the reaction liquid in the reaction container after (2) to set so that the liquid volume that has been increased by adding the diluting solution, can be brought back to the initial liquid volume,
(4) an information processing means for setting to an optimum temperature for reaction after (3) so as to restart the synthetic reaction, and
(5) an information processing means for repeating (1) to (4) for a plurality of times,
or,
(1) an information processing means for setting the synthesis time within a range of an initial phase in the synthetic reaction before the synthetic amount per unit time tends to decrease, based on information on the volume of the reaction container and the concentration of the reaction liquid,
(2) an information processing means for setting the reaction liquid out of a range of the concentration in which the synthetic reaction is substantially possible, by concentrating the reaction liquid in the reaction container when the time comes up to the synthesis time within the range of the initial phase in the synthetic reaction of (1),
(3) an information processing means for setting so as to add the diluting solution into the reaction container after (2) so that the liquid volume that has been decreased by concentrating, can be brought back to the initial liquid volume,
(4) an information processing means for setting to an optimum temperature for reaction after (3) so as to restart the synthetic reaction, and
(5) an information processing means for repeating (1) to (4) for a plurality of times.

18. A cell-free system synthesizer installed with the program according to claim 17, wherein by performing the information processing of the program jointly with the synthesizer; a means for starting the synthesis, a means for diluting/concentrating the reaction liquid, and a means for reactivating the synthetic reaction, are executed; and the synthesis system using the initial phase in the synthetic reaction having the high protein synthetic reaction velocity can be executed repeatedly for a plurality of times.

19. A protein synthesized by the synthesizer according to claims 11 to 16 and 18.
